(19) Europäisches Patentamt
European Patent Office
Office européen des brevets

(11) **EP 4 063 306 A1**

(12) **EUROPEAN PATENT APPLICATION**
published in accordance with Art. 153(4) EPC

(43) Date of publication:
**28.09.2022 Bulletin 2022/39**

(21) Application number: **20890716.2**

(22) Date of filing: **19.11.2020**

(51) International Patent Classification (IPC):
**B65H 37/04** (2006.01)  **B65H 45/12** (2006.01)
**B29C 59/04** (2006.01)  **B29C 65/08** (2006.01)

(52) Cooperative Patent Classification (CPC):
**A61F 13/15; A61F 13/511; A61F 13/512;
A61F 13/515; B29C 59/04; B29C 65/08;
B65H 37/04; B65H 45/12**

(86) International application number:
**PCT/JP2020/043287**

(87) International publication number:
**WO 2021/100832 (27.05.2021 Gazette 2021/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.11.2019  JP 2019209101**

(71) Applicant: **Kao Corporation
Chuo-ku
Tokyo 103-8210 (JP)**

(72) Inventors:
• **KURODA, Keisuke
Haga-gun, Tochigi 321-3497 (JP)**
• **MARUYAMA, Hiroshi
Haga-gun, Tochigi 321-3497 (JP)**

(74) Representative: **Vossius & Partner
Patentanwälte Rechtsanwälte mbB
Siebertstraße 3
81675 München (DE)**

(54) **METHOD AND DEVICE FOR PRODUCING COMPOSITE SHEET, COMPOSITE SHEET, AND ABSORBENT ARTICLE**

(57)  A method for producing a composite sheet of the present invention has a shape-imparting step of deforming a first sheet (1) into a projecting-and-depressed shape, a superposing step of superposing a second sheet (2) on the deformed first sheet (1), and an ultrasonic-applying step of sandwiching the superposed two sheets (1, 2) between projections (35) of a projecting-and-depressed shape roller (31) and a vibration-applying surface (42t) of a tip portion of an ultrasonic horn (42) and applying ultrasonic vibration to the sheets. A groove (46) extending along a rotating shaft of the projecting-and-depressed shape roller (31) is formed in the vibration-applying surface (42t). In the ultrasonic-applying step, through holes (6) and fused portions (4) having the through holes (6) are formed in a stack of the two sheets (1, 2) by using the ultrasonic horn (42) having the groove (46).

Fig. 6

EP 4 063 306 A1

**Description**

Technical Field

The present invention relates to a technology for producing a composite sheet in which two sheets are fused to each other and through holes are formed in fused portions.

Background Art

In a known topsheet of an absorbent article such as a disposable diaper or a sanitary napkin, projections and depressions are formed on a surface thereof that is to come into contact with the skin of a wearer.

For example, the applicant of the present invention has proposed a composite sheet that has a large number of fused portions where a first sheet and a second sheet are fused to each other, and in which portions of the first sheet other than the fused portions form projections protruding toward an opposite side to the second sheet. This composite sheet has an excellent tactile feel and an excellent ability to prevent liquid diffusion, because projections and depressions are formed on the surface.

Moreover, a configuration is also known in which through holes are formed in the fused portions of such a composite sheet to thereby improve the ability to take in liquid, and the like (see Patent Literatures 1 and 2). In Patent Literature 2, a description is also given to the effect that, in order to form the fused portions having the through holes, leading end portions of projections of a projecting-and-depressed shape roller are provided with small projections for forming openings, there being a difference in level between the small projections and shoulder portions surrounding the respective small projections, two sheets are sandwiched and heated between an anvil roller and the small projections, and thereby fused portions having openings are formed.

Citation List

Patent Literatures

Patent Literature 1: JP 2006-175688A
Patent Literature 2: JP 2006-175689A

Summary of Invention

The present invention relates to a method for producing a composite sheet that has a plurality of fused portions where a first sheet and a second sheet are fused to each other.

Preferably, in the composite sheet, at least some portions of the first sheet other than the fused portions form projections protruding toward an opposite side to the second sheet side.

Preferably, in the composite sheet, through holes are formed in the fused portions. Preferably, the production method includes a shape-imparting step of, while rotating a projecting-and-depressed shape roller that has a projecting-and-depressed shape on a peripheral surface section thereof, causing the first sheet to follow the peripheral surface section to thereby deform the first sheet into a projecting-and-depressed shape.

Preferably, the production method includes a superposing step of holding and conveying the first sheet that has been deformed into the projecting-and-depressed shape on the projecting-and-depressed shape roller and superposing the second sheet on the first sheet that is being conveyed.

Preferably, the production method includes an ultrasonic-applying step of sandwiching the superposed two sheets between projections of the projecting-and-depressed shape roller and a vibration-applying surface of a tip portion of an ultrasonic horn included in an ultrasonic fusing machine, and applying ultrasonic vibration to the sheets.

Preferably, in the ultrasonic-applying step, the through holes and the fused portions having the through holes are formed by applying ultrasonic vibration using the ultrasonic horn having a groove which is formed on the vibration-applying surface and which extends along a rotating shaft of the projecting-and-depressed shape roller.

Moreover, the present invention relates to an apparatus for producing a composite sheet that has a plurality of fused portions where a first sheet and a second sheet are fused to each other.

Preferably, in the composite sheet, at least some portions of the first sheet other than the fused portions form projections protruding toward an opposite side to the second sheet side.

Preferably, in the composite sheet, through holes are formed in the fused portions. Preferably, the production apparatus includes a projecting-and-depressed-shape-imparting unit that includes a projecting-and-depressed shape roller having a projecting-and-depressed shape on a peripheral surface section thereof and that is configured to deform the first sheet into a projecting-and-depressed shape by causing the first sheet to follow the peripheral surface section.

Preferably, the production apparatus includes an ultrasonic-applying unit that includes an ultrasonic fusing machine including an ultrasonic horn and is configured to form the through holes and the fused portions having the through holes by superposing the second sheet on the first sheet that has been deformed into the projecting-and-depressed shape, sandwiching the two sheets between projections of the projecting-and-depressed shape roller and a vibration-applying surface of a tip portion of the ultrasonic horn, and applying ultrasonic vibration to the sheets.

Preferably, a groove extending along a rotating shaft of the projecting-and-depressed shape roller is formed in the vibration-applying surface.

Moreover, the present invention relates to a composite sheet that can be used as a topsheet of an absorbent article including a liquid-retentive absorbent member, the topsheet being arranged on a side closer to the skin of a wearer than the absorbent member.

Preferably, in the composite sheet, the composite sheet has a plurality of fused portions where a first sheet and a second sheet are fused to each other, at least some portions of the first sheet other than the fused portions form projections protruding toward an opposite side to the second sheet side, and through holes are formed in the fused portions.

Preferably, in the composite sheet, protrusions are formed on a surface opposite to a surface on which the projections are formed, the protrusions starting from the plurality of fused portions, respectively, and protruding in a direction away from the second sheet.

Moreover, the present invention relates to an absorbent article having a longitudinal direction that corresponds to a front-rear direction of a wearer and a lateral direction that is perpendicular to the longitudinal direction, the absorbent article including a liquid-retentive absorbent member and a topsheet that is arranged on a side closer to the skin of the wearer than the absorbent member.

Preferably, in the absorbent article, the topsheet has a plurality of fused portions where a first sheet that forms a skin-facing surface and a second sheet that forms a non-skin-facing surface are fused to each other, at least some portions of the first sheet other than the fused portions form projections protruding toward an opposite side to the second sheet side, and through holes are formed in the fused portions.

Preferably, in the absorbent article, protrusions are formed on the non-skin-facing surface of the topsheet, the protrusions starting from the plurality of fused portions, respectively, and protruding in a direction away from the second sheet.

Other features, effects, and embodiments of the present invention will be described below.

Brief Description of Drawings

[Fig. 1] Fig. 1 is a perspective view of a relevant portion showing an example of a composite sheet produced according to the present invention.

[Fig. 2] Fig. 2 is an enlarged plan view of the composite sheet shown in Fig. 1, as viewed from a first sheet side.

[Fig. 3] Fig. 3 is a schematic diagram showing an embodiment of an apparatus for producing a composite sheet of the present invention.

[Fig. 4] Fig. 4 is a perspective view showing, in an enlarged manner, a relevant portion of a projecting-and-depressed shape roller (first roller) shown in Fig. 3.

[Fig. 5] Fig. 5 is a front view showing a relevant portion of an ultrasonic welding machine shown in Fig. 3, as viewed from an upstream side in a conveying direction of a second sheet.

[Fig. 6] Fig. 6 is a diagram showing a relevant portion (a tip portion of an ultrasonic horn and the vicinity thereof) of the production apparatus shown in Fig. 3.

[Fig. 7] Fig. 7 is an enlarged cross-sectional view schematically showing, in an enlarged manner, a cross section of the tip portion of the ultrasonic horn shown in Fig. 6, the cross section being taken along a direction (MD) that is perpendicular to a rotating shaft of the projecting-and-depressed shape roller.

[Fig. 8] Fig. 8 is a plan view of a vibration-applying surface (tip face) of the ultrasonic horn shown in Fig. 6.

[Fig. 9] Fig. 9 is a view corresponding to Fig. 7 and showing another embodiment of the ultrasonic horn according to the present invention.

[Fig. 10] Fig. 10 is a view corresponding to Fig. 7 and showing yet another embodiment of the ultrasonic horn according to the present invention.

[Fig. 11] Fig. 11 is a view corresponding to Fig. 7 and showing yet another embodiment of the ultrasonic horn according to the present invention.

[Fig. 12] Fig. 12(a) is a view corresponding to Fig. 7 and showing yet another embodiment of the ultrasonic horn according to the present invention, and Fig. 12(b) schematically shows a projecting-and-depressed shape portion shown in Fig. 12(a) and the vicinity thereof in an enlarged manner.

[Fig. 13]Fig. 13 is a schematic perspective view of an embodiment of the composite sheet of the present invention, as viewed from a surface (second sheet side) of the composite sheet opposite to a surface thereof on which projections are formed.

[Fig. 14]Fig. 14 is a cross-sectional view schematically showing a cross section taken along line I-I in Fig. 13 (a cross section taken along a direction indicated by reference sign Y and a thickness direction).

[Fig. 15]Fig. 15 is a photograph, of another embodiment of the composite sheet of the present invention, showing the surface (second sheet side) of the composite sheet opposite to the surface thereof on which projections are formed, and corresponds to Fig. 13.

[Fig. 16]Fig. 16 is an electron micrograph of a cross section of the composite sheet shown in Fig. 15, the cross section being taken along a machine direction during the production of the composite sheet and the thickness direction, and corresponds to an enlarged view of a portion (a through hole and a protrusion at a peripheral edge portion of the through hole) of Fig. 14.

[Fig. 17]Fig. 17 is a spread-out plan view schematically showing a skin-facing surface side (topsheet side) of an open type disposable diaper, which is an embodiment of an absorbent article of the present invention, in its flat-out, uncontracted state.

[Fig. 18]Fig. 18 is a horizontal cross-sectional view schematically showing a cross section taken along line II-II in Fig. 17.

[Fig. 19]Fig. 19 is a schematic cross-sectional view, taken along the thickness direction, of the topsheet of the diaper shown in Fig. 17 and a sublayer arranged on a non-skin-facing surface side of the topsheet.

[Fig. 20]Fig. 20 is a diagram showing the topsheet and the sublayer in Fig. 19 in a more schematic manner.

[Fig. 21]Fig. 21 is a view corresponding to Fig. 19 and showing another embodiment of the absorbent article of the present invention.

Description of Embodiments

If fused portions and through holes are formed in separate steps, misalignment may occur between the positions of the fused portions and the positions of the through holes. According to the method disclosed in Patent Literature 2, since the leading end portions of the projections are provided with the small projections, the fused portions and the through holes can be formed in one step. However, this method still has room for improvement in that, for example, the small projections are likely to wear out, and the apparatus maintenance load is high.

Moreover, depending on the material used to form the composite sheet, it may be difficult to form through holes. For example, in the case where constituent fibers of the composite sheet are made of a resin having a relatively high melting point, if treatment for forming fused portions and treatment for forming through holes are simultaneously performed on the composite sheet, through holes may not be formed as designed in the treated portions. For example, the resin may remain in portions where through holes are to be formed, while maintaining the form of fibers, and consequently through holes smaller than design dimensions may be formed. There is demand for a technology that enables the formation of fused portions and the formation of through holes as designed to be performed in one step irrespective of the material used to form the composite sheet.

The present invention relates to the provision of a technology that can solve the shortcomings of the conventional art described above, and more particularly to the provision of a method and an apparatus for producing a composite sheet with which, during the production of a composite sheet in which through holes are formed in fused portions, the fused portions and the through holes can be formed in one step.

Hereinafter, the present invention will be described based on preferred embodiments thereof, with reference to the drawings. In the drawings described below, the same or similar portions are denoted by the same or similar reference numerals. The drawings are basically schematic, and the ratios and the like of various dimensions shown may be different from those of actual dimensions.

First, a composite sheet that is produced using a method or an apparatus for producing a composite sheet of the present invention will be described. A composite sheet 10 has a large number of fused portions 4 where a first sheet 1 and a second sheet 2 are fused to each other.

In the composite sheet 10, at least some portions of the first sheet 1 other than the fused portions 4 form projections 5 protruding toward the opposite side to the second sheet 2 side.

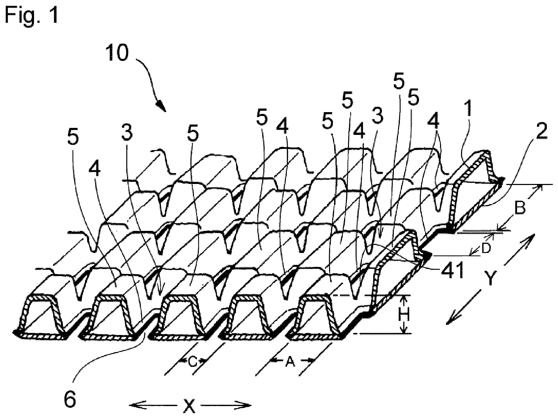

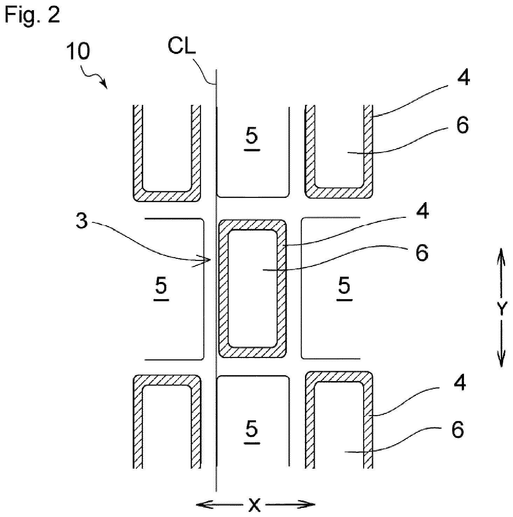

Figs. 1 and 2 show the composite sheet 10 serving an example of the composite sheet, and the composite sheet 10 has the above-described configuration.

The projections 5 and the fused portions 4 are arranged alternately in multiple rows extending in a direction X that is a direction parallel to the surface of the composite sheet 10, and the multiple rows are formed side-by-side in a direction Y that is another direction parallel to the surface of the composite sheet 10 and is perpendicular to the direction X. In adjacent rows, the projections 5 are arranged offset with respect to each other in the direction X, and the fused portions 4 are arranged offset with respect to each other in the direction X. More specifically, they are arranged offset by one-half pitch.

In the composite sheet 10, the direction Y is a direction parallel to a machine direction (hereinafter also referred to as "MD") during the production process, and the direction X is a direction parallel to a direction (hereinafter also referred to as "CD") that is perpendicular to the MD during the production process. Moreover, rotating shafts of a projecting-and-depressed shape roller 31 (first roller) and a projecting-and-depressed shape roller 32 (second roller), which will be described later, are parallel to the CD and perpendicular to the MD

The first sheet 1 and the second sheet 2 are constituted by sheet materials. For example, fiber sheets such as a nonwoven fabric, a woven fabric, and a knitted fabric, films, and the like can be used as the sheet materials. From the viewpoint of the tactile feel and the like, it is preferable to use a fiber sheet, and it is particularly preferable to use a nonwoven fabric. The sheet materials constituting the first sheet 1 and the second sheet 2 may be of the same type or of different types.

When a nonwoven fabric is used as the sheet material constituting the first sheet 1 and/or the sheet 2, examples of the nonwoven fabric include an air-through nonwoven fabric, a spunbonded nonwoven fabric, a spunlaced nonwoven fabric, a melt-blown nonwoven fabric, a resin-bonded nonwoven fabric, a needle-punched nonwoven fabric, and the like. A laminate in which two or more of these nonwoven fabrics are combined and a laminate in which any of these nonwoven fabrics is combined with a film or the like may also be used.

The basis weight of a nonwoven fabric used as the sheet materials constituting the first sheet 1 and the second sheet 2 is preferably 10 g/m$^2$ or greater, and more preferably 15 g/m$^2$ or greater.

Also, the basis weight is preferably 40 g/m$^2$ or less, and more preferably 35 g/m$^2$ or less.

Fibers made of various types of thermoplastic resins can be used as fibers that constitute the nonwoven fabric. In the case a sheet material other than a nonwoven fabric is used as well, those whose constituent fibers or constituent resins are made of various types of thermoplastic resins are favorably used.

Examples of the thermoplastic resins include polyolefins, such as polyethylene, polypropylene, and polybutene; polyesters, such as polyethylene terephthalate and polybutylene terephthalate; polyamides, such as nylon 6 and nylon 66; polyacrylic acid; polymethacrylic acid alkyl ester; polyvinyl chloride; polyvinylidene chloride; and the like. These resins may be used alone or as a blend of two or more thereof. Moreover, these resins may be used in the form of composite fibers of a core-sheath type, a side-by-side type, or the like.

Note that, in the case where a resin that forms the constituent fibers of the sheet material (nonwoven fabric) constituting the first sheet 1 and/or the second sheet 2 has a high melting point, the amount of energy that is required to melt the two sheets 1 and 2 to form the through holes 6 is higher than that in the case where the resin has a low melting point, and therefore, it may be difficult to form the through holes 6 by simply performing ultrasonic treatment in one step. Specifically, this is the case where, for example, the melting point of a resin that forms the constituent fibers is higher than 200°C, and an example of this resin is polyethylene terephthalate (PET). However, according to the present invention, due to the features thereof that will be described later, even when the material used to form the composite sheet 10 includes such a resin having a relatively high melting point, fused portions and through holes can be formed by performing ultrasonic treatment in one step.

The composite sheet 10 has a large number of depressions 3 on a first sheet 1-side surface thereof, the depressions 3 being located between the projections 5 both in the direction X and in the direction Y, and a fused portion 4 having a through hole 6 is formed at the bottom of each depression 3.

When the composite sheet 10 is viewed as a whole, the first sheet 1-side surface of the composite sheet 10 has a sharp proj ecting-and-depressed shape constituted by the depressions 3 and the projections 5, while a second sheet 2-side surface of the composite sheet 10 is flat, or is a substantially flat surface that is relatively gently undulating compared with the first sheet 1-side surface.

The individual fused portions 4 in the composite sheet 10 have substantially oblong shapes elongated in the direction Y in a plan view, and a through hole 6 having a substantially oblong shape in a plan view is formed inside each fused portion 4. In other words, the individual fused portions 4 are formed in annular shapes surrounding the respective through holes 6.

It is preferable that only a single through hole 6 is formed in a single fused portion 4, and it is preferable that the through hole 6 is formed at a predetermined specific position relative to the position of the fused portion 4.

The shape of the through hole 6 in a plan view and the shape of the outer circumferential edge of the fused portion 4 in a plan view may or may not be similar figures, but it is preferable that these shapes are similar figures.

In the fused portions 4, the first sheet 1 and the second sheet 2 are bonded to each other as a result a heat-fusible resin that forms at least one of the first and second sheets 1 and 2 being melted and solidified.

In the case where the first sheet 1 and the second sheet 2 are constituted by fiber sheets such as nonwoven fabrics, it is preferable that, in the fused portions 4, the constituent fibers of the first sheet 1 and the second sheet 2 are melted, or embedded in a molten resin, and the fibrous form thereof can no longer be visually observed, or in other words, the constituent fibers are in a filmed state in appearance. That is to say, it is preferable that the fused portions 4 are in film form.

Next, the method and the apparatus for producing a composite sheet of the present invention will be described. With the method for producing a composite sheet using a production apparatus 20, the above-described composite sheet 10 is produced.

The production apparatus 20 includes at least a projecting-and-depressed-shape-imparting unit 30 and an ultrasonic-applying unit 40.

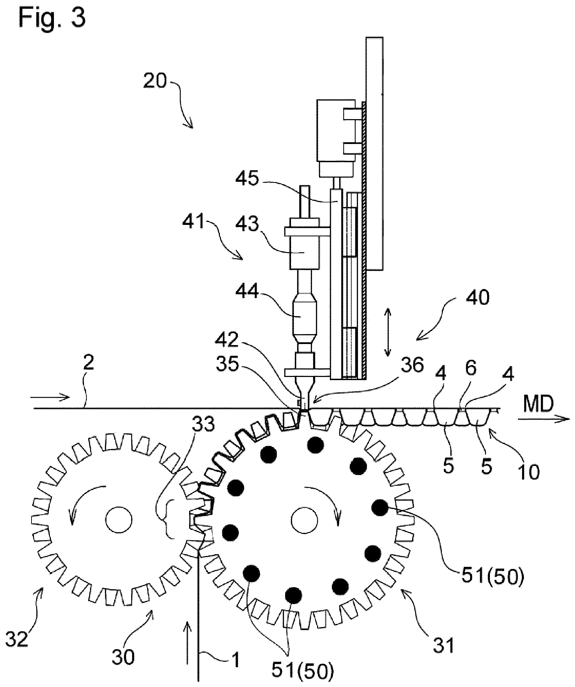

Fig. 3 shows the production apparatus 20 serving as an embodiment of the apparatus for producing a composite sheet of the present invention, and the production apparatus 20 has the above-described configuration.

The projecting-and-depressed-shape-imparting unit 30 includes a projecting-and-depressed shape roller 31 that has a projecting-and-depressed shape on a peripheral surface section thereof. In the projecting-and-depressed-shape-imparting unit 30, the first sheet 1 is made to follow the peripheral surface section of the projecting-and-depressed shape roller 31 that is rotating, and the first sheet 1 is thus deformed into a projecting-and-depressed shape that conforms to the projecting-and-depressed shape on the peripheral surface section.

In addition to the projecting-and-depressed shape roller 31, the projecting-and-depressed-shape-imparting unit 30 further includes another projecting-and-depressed shape roller 32 that has a projecting-and-depressed shape on a peripheral surface section thereof, the projecting-and-depressed shape being engageable with the projecting-and-depressed shape of the projecting-and-depressed shape roller 31.

Hereinafter, the projecting-and-depressed shape roller 31 may also be referred to as the "first roller", and the projecting-and-depressed shape roller 32 may also be referred to as the "second roller".

The projecting-and-depressed-shape-imparting unit 30 shown in Fig. 3 uses the two rollers 31 and 32, and deforms the first sheet 1 into a projecting-and-depressed shape that conforms to the projecting-and-depressed shape on the peripheral surface section of the projecting-and-depressed shape roller 31 by rotating the two rollers 31 and 32 such that an engagement portion 33 between the projecting-and-depressed shapes of the respective rollers 31 and 32 is formed, and introducing the first sheet 1 into the engagement portion 33.

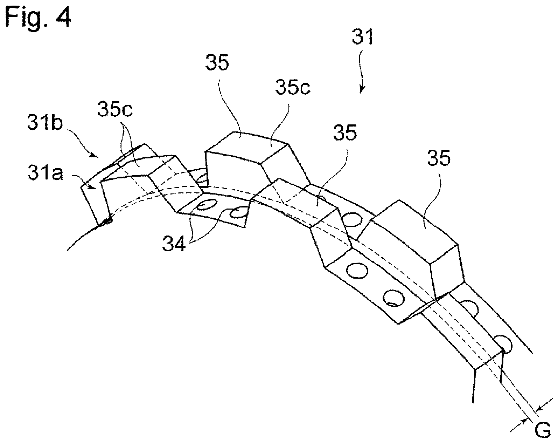

Fig. 4 shows a portion of the peripheral surface section of the projecting-and-depressed shape roller 31 (first roller).

The projecting-and-depressed shape roller 31 is formed by combining a plurality of spur gears 31a, 31b, ... having a predetermined face width into the form of a roller. Teeth of the gears form projections 35 of the projecting-and-depressed shape on the peripheral surface section of the projecting-and-depressed shape roller 31, and leading end faces 35c of the projections 35 constitute pressure-applying surfaces that apply pressure to the first and second sheets 1 and 2 that are to be fused to each other, between a vibration-applying surface 42t constituted by a tip face of an ultrasonic horn 42 of an ultrasonic fusing machine 41, which will be described later, and the pressure-applying surfaces themselves.

The face width (length in the axial direction of the gears) of the teeth of the gears constituting the projecting-and-depressed shape roller 31 determines the length of the projections 5 of the composite sheet 10 in the direction X, and the length (length in the rotating direction of the gears) of the teeth of the gears determines the length of the projections 5 of the composite sheet 10 in the direction Y.

Adjacent gears are combined such that the teeth of one of the gears are offset, by one-half tooth pitch, with respect to the teeth of the other gear. As a result, the peripheral surface section of the projecting-and-depressed shape roller 31 has the projecting-and-depressed shape.

In the form shown in Fig. 4, the leading end faces 35c of the projections 35 each have a rectangular shape with long sides extending in the rotating direction of the projecting-and-depressed shape roller 31 and short sides extending in the axial direction thereof.

It is preferable that the leading end faces 35c each have a shape that is longer in the rotating direction, because the time for which each projection 35 of the projecting-and-depressed shape roller 31 is in contact with the vibration-applying surface 42t of the tip portion of the ultrasonic horn 42 can be increased, and a temperature increase can thus be easily achieved.

Recesses of the gears of the projecting-and-depressed shape roller 31 form depressions of the projecting-and-depressed shape on the peripheral surface section of the projecting-and-depressed shape roller 31.

Suction holes 34 are formed in the bottom lands (the bottom of the recesses) of the gears. The suction holes 34 are in communication with a suction source (not shown) such as a blower or a vacuum pump, and control is performed such that suction is applied between the engagement portion 33 where the projecting-and-depressed shape rollers 31 and 32 engage with each other and a portion where the first sheet 1 joins the second sheet 2.

Accordingly, the first sheet 1 that has been deformed into a projecting-and-depressed shape by the engagement between the projecting-and-depressed shape rollers 31 and 32 is conveyed to the portion where the first sheet 1 joins the second sheet 2 and to an ultrasonic-vibration-applying portion 36 where the ultrasonic fusing machine 41 applies ultrasonic vibration to the sheets, while a suction force applied through the suction holes 34 maintains the first sheet 1 in a state in which it is deformed into the shape that conforms to the projecting-and-depressed shape on the peripheral surface section of the projecting-and-depressed shape roller 31.

In the projecting-and-depressed shape roller 31 shown in Fig. 4, a predetermined gap G is provided between adjacent gears, and this configuration suppresses problems such as an excessive stretching force being applied to the first sheet 1 or the first sheet 1 being cut in the engagement portion 33 of the two rollers 31 and 32. Therefore, the first sheet 1 can be easily deformed into the proj ecting-and-depressed shape that conforms to the shape of the peripheral surface section of the projecting-and-depressed shape roller 31.

The projecting-and-depressed shape roller 32 (second roller) has, on its peripheral surface section, the proj ecting-and-depressed shape that is engageable with the projecting-and-depressed shape on the peripheral surface section of the projecting-and-depressed shape roller 31. The projecting-and-depressed shape roller 32 has the same configuration as the projecting-and-depressed shape roller 31, except that the projecting-and-depressed shape roller 32 has no suction holes 34.

Note that the projecting-and-depressed shape roller 31 and the projecting-and-depressed shape roller 32 may have different diameters provided that the projecting-and-depressed shapes of the two rollers 31 and 32 can engage with each other. The first sheet 1 can be deformed into the projecting-and-depressed shape by introducing the first sheet 1 into the engagement portion 33 of the two rollers 31 and 32 while rotating the two rollers 31 and 32 having the projecting-and-depressed shapes that can engage with each other.

In the engagement portion 33, a plurality of portions of the first sheet 1 are pushed into the depressions on the peripheral surface section of the proj ecting-and-depressed shape roller 31 by the projections of the projecting-and-depressed shape roller 32, and the portions pushed into the depressions form projections 5 of a composite sheet 10 to be produced.

Although a plurality of projections that can be inserted into the depressions of the projecting-and-depressed shape roller 31 are formed on the peripheral surface section of the projecting-and-depressed shape roller 32, the projecting-and-depressed shape roller 32 does not need to have projections corresponding to all the depressions of the projecting-and-depressed shape roller 31.

As described above, the projecting-and-depressed-shape-imparting unit 30 shown in Fig. 3 includes the two projecting-and-depressed shape rollers having the projecting-and-depressed shapes on their respective peripheral surface sections and is configured to deform the first sheet 1 into a projecting-and-depressed shape by rotating the two projecting-and-depressed shape rollers 31 and 32 such that the engagement portion 33 between the projecting-and- depressed shapes of the two rollers 31 and 32 is formed, and introducing the first sheet 1 into the engagement portion 33. However, a configuration may also be adopted in which the projecting-and-depressed-shape-imparting unit 30 includes only the projecting-and-depressed shape roller 31 that is capable of suctioning the first sheet 1 introduced onto the peripheral surface section thereof, or in other words, the projecting-and-depressed shape roller 32 may be omitted. In this case, by simply introducing the first sheet 1 onto the peripheral surface section of the projecting-and-depressed shape roller 31, the suction force applied through the suction holes 34 (see Fig. 4) arranged in the peripheral surface section deforms the first sheet 1 so that the first sheet 1 follows the projecting-and-depressed shape of the peripheral surface section. Such following and deformation of the first sheet 1 resulting from the first sheet 1 being suctioned toward the peripheral surface section of the projecting-and-depressed shape roller 31 can be achieved by appropriately adjusting the suction force, the arrangement of the suction holes 34, and the like.

The ultrasonic-applying unit 40 includes the ultrasonic fusing machine 41 equipped with the ultrasonic horn 42, and is configured to form the through holes 6 and the fused portions 4 having the through holes 6 by superposing the second sheet 2 on the first sheet 1 that has been deformed into the projecting-and-depressed shape, sandwiching the two sheets 1 and 2 between the projections 35 of the projecting-and-depressed shape roller 31 and the vibration-applying surface 42t of the tip portion of the ultrasonic horn 42, and applying ultrasonic vibration to the sheets 1 and 2.

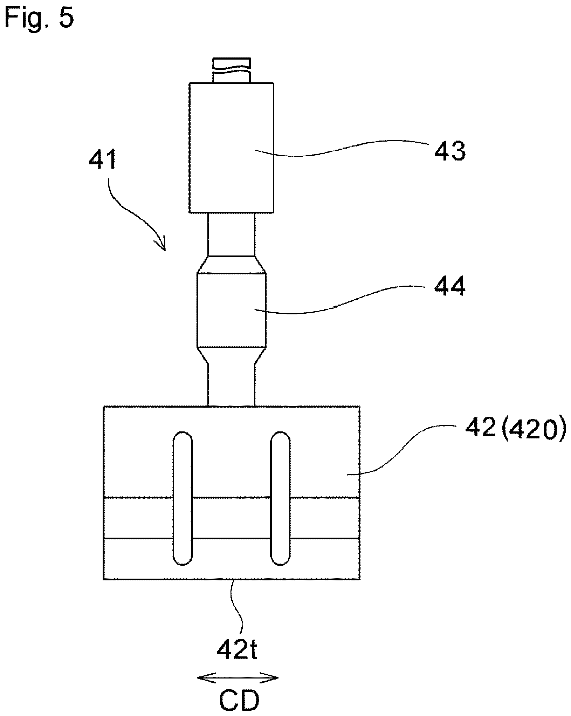

As shown in Figs. 3 and 5, the ultrasonic fusing machine 41 includes an ultrasonic oscillator (not shown), a converter 43, a booster 44, and the ultrasonic horn 42.

The ultrasonic oscillator (not shown) is electrically connected to the converter 43, and a high-voltage electric signal generated by the ultrasonic oscillator and having a wavelength corresponding to a frequency of about 15 to 50 kHz is input to the converter 43.

The ultrasonic oscillator (not shown) is installed on a movable support 45, or outside the movable support 45.

The converter 43 incorporates a piezoelectric element such as a piezoelectric element, and converts an electric signal input from the ultrasonic oscillator into mechanical vibration using the piezoelectric element. The booster 44 adjusts, preferably amplifies, the amplitude of the mechanical vibration emitted from the converter 43 and transmits the adjusted mechanical vibration to the ultrasonic horn 42.

The ultrasonic horn 42 is made of a lump of a metal such as an aluminum alloy or a titanium alloy, and is designed to correctly resonate at a frequency that is used.

The ultrasonic vibration transmitted from the booster 44 to the ultrasonic horn 42 is also amplified, or dampened, inside the ultrasonic horn 42, and then applied to the first and second sheets 1 and 2 that are to be fused to each other.

As this ultrasonic fusing machine 41, a combination of an ultrasonic horn, a converter, a booster, and an ultrasonic oscillator that are commercially available can be used.

The ultrasonic fusing machine 41 is fixed on the movable support 45, and a clearance between the vibration-applying surface 42t, which is the tip face of the ultrasonic horn 42, and the leading end faces 35c of the projections 35 of the first roller 31, as well as the applied-pressure that is applied to the superposed first and second sheets 1 and 2 can be adjusted by moving the position of the movable support 45 closer to or away from the peripheral surface section of the projecting-and-depressed shape roller 31.

While a pressure is applied to the first and second sheets 1 and 2 that are to be fused to each other sandwiched between the leading end faces 35c of the projections 35 of the projecting-and-depressed shape roller 31 and the vibration-applying surface 42t of the tip portion of the ultrasonic horn 42 of the ultrasonic fusing machine 41, ultrasonic vibration is applied to the two sheets 1 and 2. Thus, portions of the two sheets 1 and 2 that are located on the leading end faces 35c of the projections 35 generate heat, and the first sheet 1 and/or the second sheet 2 melts and then solidifies again. As a result, fused portions 4 are formed, and through holes 6 extending through the two sheets 1 and 2 are also formed in a state of being surrounded by the melted portions.

The vibration-applying surface 42t of the tip portion of the ultrasonic horn 42 is constituted by a tip face of a main body portion 420 (see Fig. 5) of the ultrasonic horn 42 made of a metal such as an aluminum alloy or a titanium alloy, and comes into contact with an object to be fused, or more specifically, the second sheet 2.

The production apparatus 20 includes a preheating means 51 for preheating at least one of the first sheet 1 and the second sheet 2 before ultrasonic vibration is applied to the sheets.

The preheating means 51 is provided inside the projecting-and-depressed shape roller 31 (first roller) and extends parallel to the rotating shaft (CD) of the projecting-and-depressed shape roller 31.

Moreover, a plurality of preheating means 51 are arranged around the rotating shaft of the projecting-and-depressed shape roller 31 and near an outer peripheral portion of the projecting-and-depressed shape roller 31, at intervals in the circumferential direction of the projecting-and-depressed shape roller 31.

Any members that can heat an object to be heated (first sheet 1 and second sheet 2) by externally applying thermal energy thereto can be used as the preheating means 51. An example thereof is a cartridge heater that uses a heating wire. However, the preheating means 51 is not limited to this, and various types of known heating means can be used without particular limitation.

The preheating means 51 is part of a preheating mechanism 50.

The preheating mechanism 50 includes, in addition to the preheating means 51, a temperature measuring means (not shown) capable of measuring the temperature of the object to be fused before ultrasonic vibration is applied thereto, and a temperature control unit (not shown) that controls the temperature of the preheating means 51 based on measured values obtained by the temperature measuring means.

The temperature to which the peripheral surface section of the projecting-and-depressed shape roller 31 is heated by the preheating means 51 is controlled by the temperature control unit. During the operation of the production apparatus 20, the preheating mechanism 50 can maintain the temperature of the first sheet 1 that is introduced into the ultrasonic-vibration-applying portion 36 within a predetermined range.

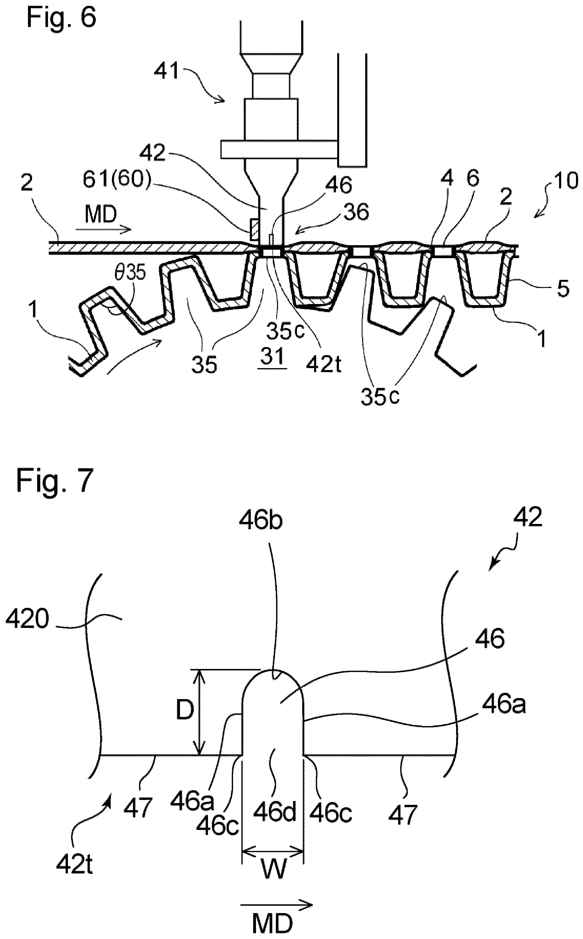

As shown in Fig. 6, the production apparatus 20 includes a horn heating means 61 for heating the ultrasonic horn 42 including the vibration-applying surface 42t.

The horn heating means 61 is not provided on the vibration-applying surface 42t, but is fixed in the vicinity of the vibration-applying surface 42t, or specifically, fixed to a side face of the tip portion of the ultrasonic horn 42.

Various types of known heating means such as a heater can be used without particular limitation as the horn heating means 61.

The horn heating means 61 is part of a horn heating mechanism 60.

The horn heating mechanism 60 includes, in addition to the horn heating means 61, a temperature measuring means (not shown) capable of measuring the temperature of the vibration-applying surface 42t, and a temperature control unit (not shown) that controls the temperature of the horn heating means 61 based on measured values obtained by the temperature measuring means.

The temperature to which the vibration-applying surface 42t is heated by the horn heating means 61 is controlled by the temperature control unit. During the operation of the production apparatus 20, the horn heating mechanism 60 can maintain the temperature of the vibration-applying surface 42t within a predetermined range.

Note that the ultrasonic fusing machine 41 applies ultrasonic vibration to the object to be fused, thereby causing the object to generate heat and melt, and consequently fusing the object, and is clearly distinguished from the above-described preheating means 51 and horn heating means 61.

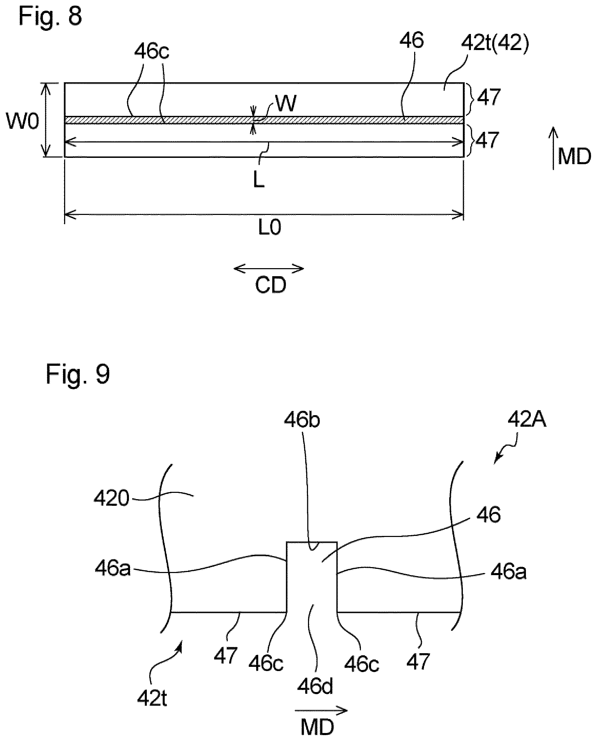

In the production apparatus 20, a groove 46 is formed in the vibration-applying surface 42t of the ultrasonic horn 42. Fig. 7 is a schematic cross-sectional view of the tip portion of the ultrasonic horn 42 taken along the MD, and Fig. 8 is a schematic plan view of the vibration-applying surface 42t of the ultrasonic horn 42. Fig. 7 is an enlarged cross-sectional view of the tip portion of the ultrasonic horn 42 shown in Fig. 6.

The groove 46 extends along the rotating shaft (CD) of the projecting-and-depressed shape roller 31 (first roller). The phrase "extends along the rotating shaft (CD)" as used herein means a case in which the angle between the groove 46 and the rotating shaft (CD) of the projecting-and-depressed shape roller 31 is less than 45°. The groove 46 shown in Fig. 8 extends parallel to the rotating shaft (CD), and the angle between the groove 46 and the rotating shaft (CD) is zero.

In the production apparatus 20, a single groove 46 is formed in the vibration-applying surface 42t. As shown in Fig. 8, the single groove 46 is located in the middle of the length of the vibration-applying surface 42t in the MD and extends over the entire length of the vibration-applying surface 42t in the CD.

In a cross-sectional view, such as that shown in Fig. 7, taken along a direction (i.e., the MD) that is perpendicular to the rotating shaft of the projecting-and-depressed shape roller 31, the groove 46 is defined by a pair of depressed sides 46a and a depressed bottom 46b.

The pair of depressed sides 46a intersect the vibration-applying surface 42t, or more specifically, are connected to the vibration-applying surface 42t and extend in a direction away from the vibration-applying surface 42t.

The depressed bottom 46b is connected to respective ends of the pair of depressed sides 46a in a longitudinal direction and opposes an opening 46d of the groove 46.

In the ultrasonic horn 42 shown in Fig. 6 (Fig. 7), corners 46c at the intersection of the vibration-applying surface 42t and the respective depressed sides 46a are sharp, and, in a cross-sectional view taken along the MD, the depressed bottom 46b has an arc shape that is curved inward away from the opening 46d.

In the form shown in Fig. 6 (Fig. 7), the angle formed by each depressed side 46a with the vibration-applying surface 42t is 90°, or in other words, the angle of each corner 46c is 90°.

A method for forming the composite sheet 10 using the production apparatus 20 that is configured as described above includes a shape-imparting step of, while rotating the proj ecting-and-depressed shape roller 31 (first roller) that has the projecting-and-depressed shape on the peripheral surface section thereof, causing the first sheet 1 to follow the peripheral surface section to thereby deform the first sheet 1 into a projecting-and-depressed shape.

Also, the method for producing the composite sheet 10 using the production apparatus 20 includes a superposing step of holding and conveying the first sheet 1 that has been deformed into the projecting-and-depressed shape on the projecting-and-depressed shape roller 31 and superposing the second sheet 2 on the first sheet 1 that is being conveyed.

Furthermore, the method for producing the composite sheet 10 using the production apparatus 20 includes an ultrasonic-applying step of sandwiching the superposed two sheets 1 and 2 between projections 35 of the projecting-and-depressed shape roller 31 and the vibration-applying surface 42t of the tip portion of the ultrasonic horn 42 included in the ultrasonic fusing machine 41, and applying ultrasonic vibration to the sheets 1 and 2.

In the shape-imparting step, the first sheet 1 is introduced into the engagement portion 33 where the projecting-and-depressed shapes of the two projecting-and-depressed shape rollers 31 and 32 engage with each other, and deformed into the projecting-and-depressed shape.

Then, in the ultrasonic-applying step, through holes 6 and fused portions 4 having the through holes 6 are formed in the stack (object to be fused) of the superposed first and second sheets 1 and 2 by applying ultrasonic vibration thereto using, as the ultrasonic horn, a specific ultrasonic horn that has been described above, that is, the ultrasonic horn 42 in which the groove 46 extending along the rotating shaft (CD) of the projecting-and-depressed shape roller 31 (first roller) is formed in the vibration-applying surface 42t.

In the ultrasonic-applying step, as shown in Fig. 6, while the object to be fused (stack of the first sheet 1 and the second sheet 2) is being conveyed in the MD, the object to be fused is sandwiched between the leading end faces 35c of the projections 35 of the projecting-and-depressed shape roller 31 and the vibration-applying surface 42t in which the groove 46 is formed, of the ultrasonic horn 42, and ultrasonic vibration is applied thereto.

Here, the vibration-applying surface 42t that presses the object to be fused against the projections 35 has, as shown in Fig. 7, the pair of corners 46c that are located in front of and behind the opening 46d of the groove 46 in the MD Therefore, stress that is generated when pressing the object to be fused is concentrated on the corners 46c, and a shear force that is applied to the object to be fused via the corners 46c is increased, compared with that in the case where the corners 46c (groove 46) are not formed. Accordingly, in the ultrasonic-applying step, in addition to heat generated in the object to be fused due to the ultrasonic vibration, a strong shear force caused by the groove 46 also acts on the object to be fused, and as a result, both the fused portions 4 and the through holes 6 can be simultaneously formed in a portion of the object to be fused that is sandwiched between the leading end faces 35c of the projections 35 and the vibration-applying surface 42t of the ultrasonic horn 42.

With the above-described ultrasonic-applying step, even when a resin that forms the first sheet 1 and/or the second sheet 2 is a resin (e.g., PET) having a high melting point that exceeds 200°C, the fused portions 4 and the through holes 6 can be formed simultaneously. Moreover, misalignment that may occur between the positions of the fused portions 4 and the positions of the through holes 6 when the fused portions 4 and the through holes 6 are formed in separate steps is prevented by forming the fused portions 4 and the through holes 6 simultaneously.

In particular, with the ultrasonic horn 42, since the corners 46c that define the opening 46d of the groove 46 are sharp as shown in Fig. 7, the shear force applied to the object to be fused (stack of the first sheet 1 and the second sheet 2) in the ultrasonic-applying step is increased, compared with that in the case where the corners 46c are rounded rather than being sharp, and therefore, the through holes 6 can be formed more easily, and the fused portions 4 and the through holes 6 can be simultaneously formed even more reliably.

From the viewpoint of ensuring that the above-described effect of the corners 46c is exhibited even more reliably, the angle between the vibration-applying surface 42t and the respective depressed sides 46a at the corners 46c is preferably 45° or greater, and more preferably 60° or greater.

Also, the above-described angle is preferably 135° or less, and more preferably 120° or less.

On the other hand, the formation of the groove 46 in the vibration-applying surface 42t of the ultrasonic horn 42 reduces the durability of the ultrasonic horn 42 (in particular, the main body portion 420), and thus, there is concern that a crack (crevice) starting from the groove 46 may form in the main body portion 420 and the like during the application of ultrasonic vibration. However, in the ultrasonic horn 42, this concern is eliminated by forming the depressed bottom 46b, which defines the groove 46, into an arc shape that is curved inward away from the opening 46d in a cross-sectional view, such as that shown in Fig. 7, of the ultrasonic horn 42 taken along the MD.

From the viewpoint of ensuring that the above-described effect of the depressed bottom 46b is exhibited even more reliably, the curvature of the depressed bottom 46b is preferably 1 or greater, and more preferably 2 or greater.

Also, the curvature of the depressed bottom 46b is preferably 10 or less, and more preferably 5 or less.

From the viewpoint of ensuring that the above-described effect of the groove 46 is exhibited even more reliably, it is preferable that the dimensions and the like of the groove 46 are set as follows.

The width W (see Figs. 7 and 8) of the groove 46 is preferably 0.2 mm or greater, and more preferably 0.5 mm or greater.

Also, the width W is preferably 2 mm or less, and more preferably 1 mm or less.

The width W0 (see Fig. 8) of the vibration-applying surface 42t is preferably 5 mm or greater, and more preferably 10 mm or greater.

Also, the width W0 is preferably 20 mm or less, and more preferably 15 mm or less.

The ratio of the length of the groove 46 in the CD, that is, the length L (see Fig. 8) of the groove 46 along the rotating shaft of the projecting-and-depressed shape roller 31 (first roller) to the length L0 (see Fig. 8) of the vibration-applying surface 42t in the same direction, length L/length L0, is preferably 0.2 or greater, and more preferably 0.3 or greater.

Also, the above-described ratio (length L/length L0) is preferably 1 or less.

In the form shown in Fig. 8, the groove 46 extends over the entire length of the vibration-applying surface 42t in the CD. This means that the length L and the length L0 are equal, and the above-described ratio is 1.

The length L0 of the vibration-applying surface 42t in the CD is preferably 30 mm or greater, and more preferably 50 mm or greater.

Also, the length L0 is preferably 200 mm or less, and more preferably 150 mm or less.

The depth D (see Fig. 7, the length from the vibration-applying surface 42t to a portion of the depressed bottom 46b that is the farthest from the vibration-applying surface 42t) of the groove 46 is preferably 0.3 mm or greater, and more preferably 0.5 mm or greater.

Also, the depth D is preferably 5 mm or less, and more preferably 2 mm or less.

It is preferable that the groove 46 is formed in a middle portion of the vibration-applying surface 42t in the MD, and it is particularly preferable that the groove 46 is formed in a region preferably within 5 mm, and more preferably within 3 mm, from the middle of the vibration-applying surface 42t in the MD toward the upstream side in the MD

In the form shown in Fig. 8, the groove 46 is formed in the middle of the vibration-applying surface 42t in the MD.

In the ultrasonic-applying step, the applied-pressure that is applied to the first and second sheets 1 and 2 sandwiched between the leading end faces 35c of the projections 35 of the projecting-and-depressed shape roller 31 (first roller) and the vibration-applying surface 42t of the ultrasonic horn 42 is preferably 20 N/mm or greater, and more preferably 30 N/mm or greater, from the viewpoint of the ease of forming the fused portions 4 and the through holes 6.

Also, the applied-pressure is preferably 80 N/mm or less, and more preferably 70 N/mm or less.

The "applied-pressure" as used herein refers to so-called "linear load", and is indicated by the value (applied-pressure per unit length) obtained by dividing the applied-pressure (N) applied from the ultrasonic horn 42 by the total length (excluding the depressions of the projecting-and-depressed shape roller 31) of the face widths (lengths of the projections 35 in the CD) of the projections 35 that are in contact with the ultrasonic horn 42.

From the same viewpoint, the frequency of ultrasonic vibration that is applied is preferably 15 kHz or higher, and more preferably 20 kHz or higher.

Also, the frequency is preferably 50 kHz or lower, and more preferably 40 kHz or lower.

From the same viewpoint, the amplitude of ultrasonic vibration that is applied is preferably 20 $\mu$m or greater, and more preferably 25 $\mu$m or greater.

Also, the amplitude is preferably 50 $\mu$m or less, and more preferably 40 $\mu$m or less.

To measure the frequency and amplitude of ultrasonic vibration, displacement of the tip of the ultrasonic horn is measured using a laser displacement meter or the like, and the frequency and amplitude are measured by setting the sampling rate to 200 kHz or higher and the precision to 1 $\mu$m or greater.

As described above, the production apparatus 20 includes the preheating means 51 (preheating mechanism 50), and, in the method for producing the composite sheet 10 using the production apparatus 20, at least one of the first sheet 1 and the second sheet 2 is preheated by the preheating means 51 before the sheets are subjected to the ultrasonic-applying step. This, combined with the effect of the groove 46, enables even more reliable simultaneous formation of the fused portions 4 and the through holes 6.

The conditions for preheating the object to be fused using the preheating means 51 are not particularly limited, and can be appropriately adjusted according to the type and the like of the object to be fused. However, it is preferable that at least one of the first sheet 1 and the second sheet 2 is heated in advance to a temperature that is below the melting point of the sheet but is not below a temperature that is 50°C lower than the melting point. That is to say, it is preferable to perform one or both of (1) and (2) below before the application of ultrasonic vibration.

(1) The first sheet 1 is heated in advance to a temperature that is below the melting point of the first sheet but is not below a temperature that is 50°C lower than the melting point.
(2) The second sheet 2 is heated in advance to a temperature that is below the melting point of the second sheet but is not below a temperature that is 50°C lower than the melting point.

Preferably, the first sheet 1 is heated in advance to a temperature that is below the melting point of the first sheet but is not below a temperature that is 50°C lower than the melting point, and the second sheet 2 is also heated in advance to a temperature that is below the melting point of the second sheet but is not below a temperature that is 50°C lower than the melting point.

As the method for (1) above, that is, the method for heating the first sheet 1 to a temperature that is below the melting point of the first sheet 1 but is not below a temperature that is 50°C lower than the melting point, for example, the temperature of the first sheet 1 on the projecting-and-depressed shape roller 31 (first roller) is measured between the engagement portion 33 of the projecting-and-depressed shape rollers 31 and 32 and the ultrasonic-vibration-applying portion 36 of the ultrasonic fusing machine 41, and the temperature of the preheating means 51 is controlled such that measured values are within the above-described specific range.

As the method for preheating the first sheet 1 to a temperature within the specific range, various other methods can be used, instead of the method in which the temperature of the peripheral surface section of the projecting-and-depressed shape roller 31 is controlled by a heater, the heater being provided inside the projecting-and-depressed shape roller 31, such that the temperature of the first sheet 1 is within the specific range.

Examples of the other methods include: a method in which a heater, a hot-air supply port, or a far-infrared irradiation device is provided near the peripheral surface section of the projecting-and-depressed shape roller 31, and the temperature of the peripheral surface section of the projecting-and-depressed shape roller 31 before or after the first sheet 1 is applied thereto is controlled using the heater, the hot-air supply port, or the far-infrared irradiation device; and a method in which the projecting-and-depressed shape roller 32 (second roller) that comes into contact with the first sheet 1 in the engagement portion 33 is heated, and the temperature of the first sheet 1 is controlled by controlling the temperature of the peripheral surface section of the projecting-and-depressed shape roller 32.

Other examples include: a method in which the first sheet 1 before being applied to the projecting-and-depressed shape roller 31 is brought into contact with a heated roller, is passed through a space in which a high temperature is maintained, or is exposed to hot air sprayed thereon; and the like.

As the method for (2) above, that is, the method for heating the second sheet 2 to a temperature that is below the melting point of the second sheet 2 but is not below a temperature that is 50°C lower than the melting point, it is preferable to measure the temperature of the second sheet 2 before the second sheet 2 joins the first sheet 1 with use of a temperature measuring means arranged on a conveyance path of the second sheet 2, and control the temperature of a heating means (not shown) for the second sheet 2, the heating means being arranged on the conveyance path of the second sheet 2, such that measured values are within the above-described specific range.

The heating means for the second sheet 2 may be of a contact type that, for example, brings a heated roller or the like into contact with the second sheet 2, or may be of a contactless type that, for example, passes the second sheet 2 through a space in which a high temperature is maintained, sprays hot air onto the second sheet 2 or passes hot air through the second sheet 2, or irradiates the second sheet 2 with infrared rays.

The melting points of the first sheet 1 and the second sheet 2 can be measured using a differential scanning calorimeter (DSC), PYRIS Diamond DSC, manufactured by Perkin-Elmer, for example. In this measurement method, the melting points of the measurement targets (the first sheet 1 and the second sheet 2) are determined from peak values of measurement data.

In the case where the first sheet 1 or the second sheet 2 is a fiber sheet such as a nonwoven fabric, and the constituent fibers thereof are composite fibers composed of a plurality of components, such as composite fibers of a core-sheath type or a side-by-side type, the melting point of that sheet is defined as follows: the lowest melting point of a plurality of melting points measured using the DSC is used as the melting point of the composite fiber sheet.

Moreover, as described above, the production apparatus 20 includes the horn heating means 61 (horn heating mechanism 60), and, in the ultrasonic-applying step, the vibration-applying surface 42t heated by the horn heating means 61 is brought into contact with the object to be fused (stack of the first sheet 1 and the second sheet 2). This, combined with the effect of the groove 46, enables even more reliable simultaneous formation of the fused portions 4 and the through holes 6.

The conditions for heating using the horn heating means 61 are not particularly limited, and can be appropriately adjusted according to the type and the like of the object to be fused.

For example, the method for (2) above may be performed by using the horn heating means 61 instead of the preheating means 51. More specifically, the following method may be performed: the temperature of the ultrasonic horn 42 (vibration-applying surface 42t) heated by the horn heating means 61 is controlled, thereby the temperature of the second sheet 2 immediately before the application of ultrasonic vibration is heated to a temperature that is below the melting point of the second sheet 2 but is not below a temperature that is 50°C lower than the melting point, and in this state, ultrasonic vibration is applied to the first and second sheets 1 and 2 sandwiched between the projections 35 of the projecting-and-depressed shape roller 331 and the vibration-applying surface 42t.

Furthermore, only one of the preheating means 51 and the horn heating means 61 may be used, or both of these means may be used together.

Figs. 9 to 12 show a relevant portion (tip portion) of other embodiments of the ultrasonic horn according to the present invention.

For the embodiments described below, constituent portions different from those of the above-described ultrasonic horn 42 are mainly described, while constituent portions similar to those of the ultrasonic horn 42 are denoted by the same reference numerals, and descriptions thereof are omitted. For constituent portions that will not be specifically described, descriptions with respect to the ultrasonic horn 42 apply as appropriate.

In an ultrasonic horn 42A shown in Fig. 9, in a cross-sectional view of the ultrasonic horn 42 taken along the MD, such as that shown in Fig. 9, the depressed bottom 46b of the groove 46 is straight line-shaped, and the groove 46 has an oblong shape in the same cross-sectional view. That is to say, the depressed bottom 46b of the ultrasonic horn 42A is flat.

In the case where the ultrasonic horn 42A is used, basically, similar effects to the effects in the case where the above-described ultrasonic horn 42 is used can be exhibited. However, from the viewpoint of even more reliably suppressing problems such as the above-described reduction in the durability of the ultrasonic horn 42 and the resulting cracking, which may occur as a result of the groove 46 being formed, it is preferable that the shape of the depressed bottom 46b in the above-described cross-sectional view is an arc shape that is curved inward away from the opening 46d as shown in Fig. 7.

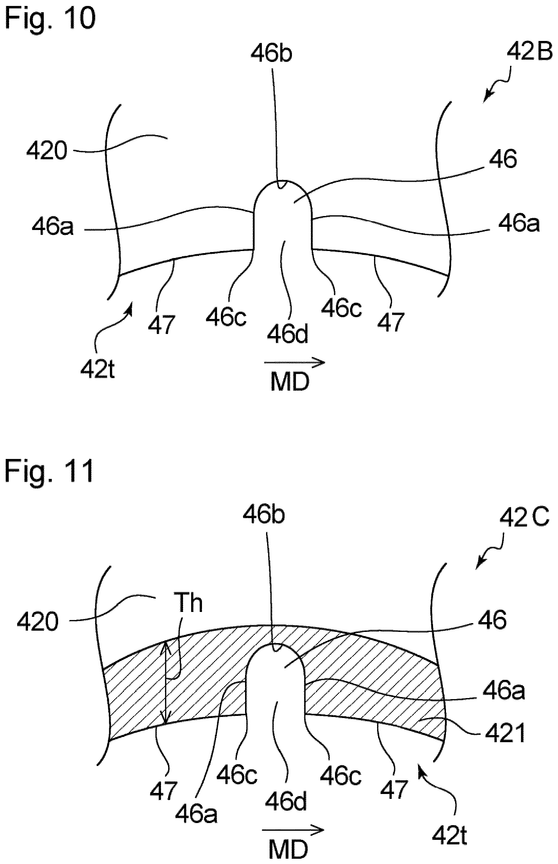

In an ultrasonic horn 42B shown in Fig. 10, in a cross-sectional view taken along the direction (MD) that is perpendicular to the rotating shaft of the projecting-and-depressed shape roller 31 (first roller), the vibration-applying surface 42t has an arc shape that is curved inward away from the rotating shaft.

Note that the "vibration-applying surface 42t" as used herein is a vibration-applying surface in the case where it is assumed that no groove 46 is present, or more specifically, it is a vibration-applying surface in the case where, in a cross-sectional view, such as that shown in Fig. 10, taken along the MD, the vibration-applying surface 42t is imaginarily extended from the corner 46c on one side of the opening 46d of the groove 46 in the MD to the corner 46c on the other side.

The vibration-applying surface 42t that has an arc-shape in cross section taken along the MD as described above increases the shear force applied to the object to be fused (stack of the first sheet 1 and the second sheet 2) in the ultrasonic-applying step. This, combined with the effect of the groove 46, enables even more reliable simultaneous formation of the fused portions 4 and the through holes 6.

It is preferable that the vibration-applying surface 42t having an arc shape in a cross-sectional view, such as that shown in Fig. 10, taken along the MD, is curved along a circular trajectory (not shown) of the leading ends of the projections 35 of the projecting-and-depressed shape roller 31 (first roller). This configuration increases the time for which the object to be fused (stack of the first sheet 1 and the second sheet 2) is sandwiched between the leading end faces 35c of the projections 35 and the vibration-applying surface 42t and therefore enables even more reliable simultaneous formation of the fused portions 4 and the through holes 6.

Moreover, in the case where the vibration-applying surface 42t of the ultrasonic horn 42 has an arc shape in a cross-sectional view taken along the MD as described above, it is preferable that the leading end face 35c of each of the plurality of projections 35 of the projecting-and-depressed shape roller 31 corresponding to this vibration-applying surface 42t has a convex shape that is curved outward away from the rotating shaft of the projecting-and-depressed shape roller 31 in the same cross-sectional view, and the curvature direction of the leading end face 35c is the same as that of the vibration-applying surface 42t.

The radius of curvature of the vibration-applying surface 42t of the ultrasonic horn 42B is preferably 100% or greater of the radius of curvature of the leading end face 35c of each projection 35 of the projecting-and-depressed shape roller 31.

Also, the radius of curvature of the vibration-applying surface 42t is preferably 500% or less, and more preferably 200% or less, of the radius of curvature of the leading end face 35c of each projection 35.

Note that, although the vibration-applying surface 42t of the ultrasonic horn 42B shown in Fig. 10 has an arc shape in cross section taken along the MD, over the entire region thereof in the direction that is parallel to the rotating shaft of the projecting-and-depressed shape roller 31, the vibration-applying surface 42t may also have a portion with a different cross-sectional shape in, for example, a region thereof that does not oppose the projections 35, in the direction that is parallel to the rotating shaft.

For example, in the case where a gap G is provided between adjacent gears constituting the projecting-and-depressed shape roller 31 as shown in Fig. 4, a flat portion or the like that does not protrude from the arc-shaped vibration-applying surface 42t may be provided in a region of the vibration-applying surface 42t that opposes the gap G.

In an ultrasonic horn 42C shown in Fig. 11, the tip portion of the ultrasonic horn 42 includes a heat storage portion 421 fixed to the main body portion 420 of the ultrasonic horn 42C, the main body portion 420 being made of a metal, and the vibration-applying surface 42t is formed of the heat storage portion 421.

The groove 46 is formed in at least the heat storage portion 421.

In Fig. 11, the groove 46 is formed in only the heat storage portion 421. However, the groove 46 may extend through the heat storage portion 421 in the thickness direction and extend into the main body portion 420.

Moreover, as with the vibration-applying surface 42t of the above-described ultrasonic horn 42B, the vibration-applying surface 42t formed of the heat storage portion 421 shown in Fig. 11 has an arc shape in a cross-sectional view taken along the MD However, the vibration-applying surface 42t may be flat without having an arc shape.

The heat storage portion 421 is made of a heat storage material that is a material having a lower thermal conductivity than the metal that forms the main body portion 420.

The thermal conductivity of the heat storage material that forms the heat storage portion 421 is preferably 2.0 W/mK or less, and more preferably 1.0 W/mK or less, from the viewpoint of reducing heat dissipation to the ultrasonic horn and the atmosphere.

Also, the thermal conductivity of the heat storage material is preferably 0.1 W/mK or greater, and more preferably 0.5 W/mK or greater, from the viewpoint of efficiently heating the sheets.

The thermal conductivity of the heat storage material can be measured in accordance with a common method using a thermal conductivity measuring apparatus.

When the vibration-applying surface 42t is formed of the heat storage portion 421, heat from the first and second sheets 1 and 2 that have generated heat due to ultrasonic vibration is stored in the heat storage portion 421, and consequently the temperature of the heat storage portion 421 increases and the first and second sheets 1 and 2 can thus be heated. This, combined with the effect of the groove 46 formed in the vibration-applying surface 42t, enables even more reliable simultaneous formation of the fused portions 4 and the through holes 6.

Also, when the vibration-applying surface 42t is formed of the heat storage portion 421, the occurrence of problems such as the adhesion of a molten resin to a conveyance means, the molten resin being melted as a result of the first and second sheets 1 and 2 melting, and the wrapping of the sheets around a conveyance roller is suppressed, which has the advantage of reducing the maintenance load of the production apparatus.

The thickness Th (see Fig. 11) of the heat storage portion 421 is not particularly limited, but is preferably 5 $\mu$m or greater, and more preferably 10 $\mu$m or greater, from the viewpoint of ensuring that the effect of the heat storage portion 421 is exhibited more reliably.

Also, the thickness Th is preferably 100 $\mu$m or less, and more preferably 50 $\mu$m or less.

A synthetic resin having excellent wear resistance and heat resistance is preferably used as the heat storage material constituting the heat storage portion 421, provided that the synthetic resin has a lower thermal conductivity than the metal constituting the main body portion 420. Examples of this synthetic resin include synthetic resins having a Rockwell hardness from R120 to R140 and a heat resistance temperature from 150°C to 500°C, such as polyimide, polybenzimidazole, polyether ethyl ketone, polyphenylene sulfite, polyetherimide, and polyamideimide.

Synthetic resins having a Rockwell hardness from R125 to R140 and a heat resistance temperature from 280°C to 400°C, such as polyimide and polybenzimidazole, are particularly preferable as the heat storage material.

Here, the Rockwell hardness is a value measured in conformity with ASTM D-785, and the heat resistance temperature is a value measured in conformity with ASTM D-648.

There is no particular limitation on the means for fixing the heat storage portion 421 made of a synthetic resin to the main body portion 420 made of a metal, and a known fixing means can be employed.

The heat storage portion 421, which is made of a synthetic resin, can be fixed to the main body portion 420, which is made of a metal, by, for example, forming the heat storage portion 421 on the main body portion 420 through thermal spraying.

The term "thermal spraying" as used herein refers to a known surface treatment process of forming a coating film on a base material surface by accelerating particles of a thermal spraying material, such as a metal or a ceramic, that has been melted or nearly melted through heating, and allowing the particles to impinge on the base material surface at a high speed.

As the thermal spraying material, any material that can be thermal-sprayed and can contribute to improvement of the fixing strength of the heat storage portion 421, which is made of a synthetic resin, can be used without particular limitation. However, ceramics, such as tungsten carbide, zirconia, and chromium carbide; alloys, such as an aluminum-magnesium alloy and a zinc-aluminum alloy; metals, such as aluminum, stainless steel, titanium, and molybdenum; a cermet, which is a composite material composed of a metal and a ceramic; and the like can be favorably used, in view of their excellent ability to bind to the main body portion 420 made of a metal such as a titanium alloy and also their excellent wear resistance and heat resistance.

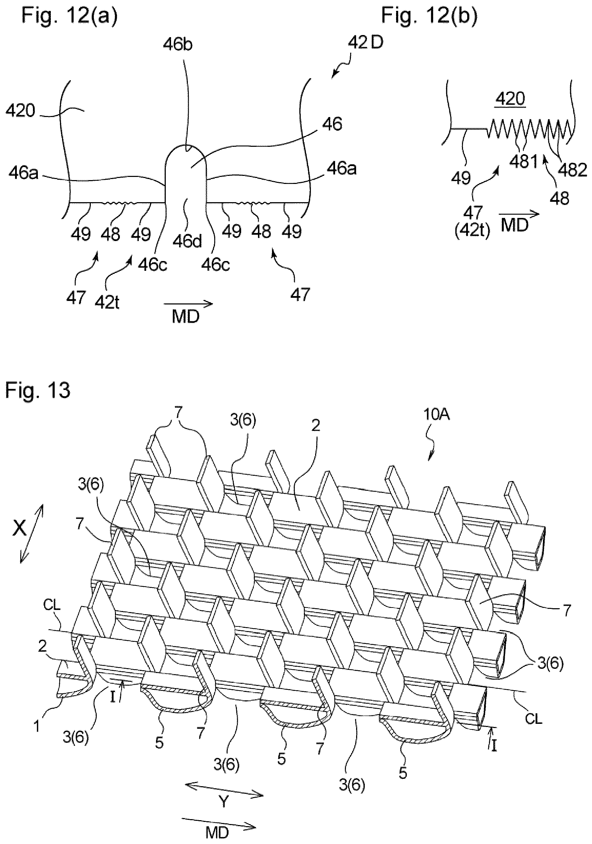

In an ultrasonic horn 42D shown in Fig. 12, a projecting-and-depressed shape portion 48 is formed in a groove-less portion 47 of the vibration-applying surface 42t.

More specifically, as shown in Fig. 12(a), part of the groove-less portion 47 is the projecting-and-depressed shape portion 48, and the remaining part of the groove-less portion 47 is a smooth portion 49 that is smooth without projections and depressions. The projecting-and-depressed shape portion 48 has a larger surface roughness, and therefore a stronger frictional force, than the smooth portion 49.

In the ultrasonic-applying step, a shear force acts on a portion of the object to be fused (stack of the first sheet 1 and the second sheet 2) that is pressed by the projecting-and-depressed shape portion 48. This, combined with the effect of the groove 46, enables even more reliable simultaneous formation of the fused portions 4 and the through holes 6.

As shown in Fig. 12(b), the projecting-and-depressed shape portion 48 has a plurality of projections 481 and a plurality of depressions 482. The projections 481 have a triangular shape in a cross-sectional view, such as that shown in Fig. 12(b), taken along the MD, but the shape of the projections 481 in this cross-sectional view is not particularly limited, and may be, for example, a rectangle, a trapezoid, or the like.

Moreover, an example of the arrangement pattern of the plurality of projections 481 in the projecting-and-depressed shape portion 48 is an arrangement pattern in which projection rows in each of which projections 481 are arranged at regular intervals in the CD (direction along the rotating shaft of the projecting-and-depressed shape roller 31) are arranged at regular intervals in the MD

Another example of the arrangement pattern is an arrangement pattern in which projection rows in each of which projections 481 are arranged at regular intervals in the CD are arranged at regular intervals in the MD, and adjacent projection rows that are adjacent to each other in the MD are arranged offset by one-half pitch.

The projecting-and-depressed shape portion 48 can be formed by performing knurling or thermal spraying on the groove-less portion 47 of the vibration-applying surface 42t.

In the form shown in Fig. 12, the smooth portion 49 is present between the groove 46 and the projecting-and-depressed shape portion 48. However, a configuration may also be adopted in which no smooth portion 49 is present between the groove 46 and the projecting-and-depressed shape portion 48, and the groove 46 and the projecting-and-depressed shape portion 48 are next to each other in the MD.

Moreover, a configuration may also be adopted in which no smooth portion 49 is present on the vibration-applying surface 42t, and the entire groove-less portion 47 constitutes the projecting-and-depressed shape portion 48.

From the viewpoint of ensuring that the effect of the projecting-and-depressed shape portion 48 is exhibited even more reliably, the surface roughness of the projecting-and-depressed shape portion 48 is preferably 3.2 $\mu$m or greater, and more preferably 6.3 $\mu$m or greater, in terms of the arithmetic mean roughness Ra.

Also, the surface roughness of the projecting-and-depressed shape portion 48 is preferably 25 $\mu$m or less, and more preferably 12.5 $\mu$m or less, in terms of the arithmetic mean roughness Ra.

The arithmetic mean roughness Ra can be measured using various types of surface roughness measuring machines, and, for example, can be measured using a surface roughness measuring machine manufactured by Mitutoyo Corporation.

From the same viewpoint, the dimensions and the like of the projecting-and-depressed shape portion 48 are preferably set as follows.

The ratio of the area (48S) of the projecting-and-depressed shape portion 48 of the vibration-applying surface 42t to the area (47S) of the groove-less portion 47 thereof, that is, the ratio calculated by (48S/47S)$\times$100 is preferably 15% or greater, and more preferably 30% or greater.

Also, the above-described ratio is preferably 100% or less, and more preferably 80% or less.

The number of projections 481, which constitute the projecting-and-depressed shape portion 48, per unit area (1 cm$^2$) is preferably 1 or greater, and more preferably 100 or greater.

Also, the number of projections 481 per unit area (1 cm$^2$) is preferably 1,000,000 or less, and more preferably 10,000 or less.

In a plan view of the projecting-and-depressed shape portion 48, the area of a single projection 481 is preferably

0.0001 mm$^2$ or greater, and more preferably 0.01 mm$^2$ or greater.

Also, the area of a single projection 481 is preferably 100 mm$^2$ or less, and more preferably 1 mm$^2$ or less.

It is preferable that the composite sheet 10 produced according to the present invention has the following configurations.

The height H (see Fig. 1) of the projections 5 is preferably 1 mm or greater, and more preferably 3 mm or greater.

Also, the height H of the projections 5 is preferably 10 mm or less, and more preferably 6 mm or less.

The number of projections 5 per unit area (1 cm$^2$) in the composite sheet 10 is preferably 1 or greater, and more preferably 6 or greater.

Also, the number of projections 5 per unit area (1 cm$^2$) in the composite sheet 10 is preferably 20 or less, and more preferably 15 or less.

The bottom length A (see Fig. 1) of the projections 5 in the direction X is preferably 0.5 mm or greater, and more preferably 1.0 mm or greater.

Also, the bottom length A is preferably 5.0 mm or less, and more preferably 4.0 mm or less.

The bottom length B (see Fig. 1) of the projections 5 in the direction Y is preferably 1.0 mm or greater, and more preferably 2.0 mm or greater.

Also, the bottom length B is preferably 10.0 mm or less, and more preferably 7.0 mm or less.

The ratio of the bottom length A in the direction X to the bottom length B in the direction Y (bottom length A:bottom length B) is preferably 1:1 or greater, and more preferably 1:2 or greater.

Also, the above-described ratio (bottom length A:bottom length B) is preferably 1:10 or less, and more preferably 2:5 or less.

The bottom area (bottom length A × bottom length B) of the projections 5 is preferably 0.5 mm$^2$ or greater, and more preferably 2 mm$^2$ or greater.

Also, the bottom area of the projections 5 is preferably 50 mm$^2$ or less, and more preferably 20 mm$^2$ or less.

The length C (see Fig. 1) of the fused portions 4 in the direction X is preferably 0.5 mm or greater, and more preferably 0.8 mm or greater.

Also, the length C is preferably 2 mm or less, and more preferably 1.5 mm or less.

The length D (see Fig. 1) of the fused portions 4 in the direction Y is preferably 1.0 mm or greater, and more preferably 1.2 mm or greater.

Also, the length D is preferably 5.0 mm or less, and more preferably 3.0 mm or less.

The ratio of the length C in the direction X to the length D in the direction Y (length C:length D) is preferably 1:1 or greater, and more preferably 2:3 or greater.

Also, the above-described ratio (length C:length D) is preferably 1:3 or less, and more preferably 2:5 or less.

The area of a region inside the outer circumferential edge of each fused portion 4 is preferably 0.5 mm$^2$ or greater, and more preferably 1.0 mm$^2$ or greater.

Also, the area of the region inside the outer circumferential edge of each fused portion 4 is preferably 5.0 mm$^2$ or less, and more preferably 4.0 mm$^2$ or less.

The "area of a region inside the outer circumferential edge of each fused portion 4" as used herein contains the area of the through hole 6.

The opening area of each through hole 6 is preferably 50% or greater, and more preferably 80% or greater, of the area of the region inside the outer circumferential edge of each fused portion 4.

Also, the opening area of each through hole 6 is preferably less than 100%, and more preferably 95% or less, of the area of the region inside the outer circumferential edge of each fused portion 4.

The opening area of each through hole 6 is preferably 1 mm$^2$ or less, and more preferably 3 mm$^2$ or less.

Also, the opening area of each through hole 6 is preferably 10 mm$^2$ or less, and more preferably 7 mm$^2$ or less.

The composite sheet 10 has a projecting-and-depressed shape, and, in addition, has the fused portions 4 having the through holes 6, at the bottom of the depressions 3 constituting the projecting-and-depressed shape. Therefore, the composite sheet 10 has an excellent tactile feel and an excellent ability to prevent liquid diffusion in a plane direction, and also has excellent air permeability and an excellent ability to take in liquid.

Taking advantage of the above-described properties, the composite sheet 10 is favorably used as a constituent member of an absorbent article such as a disposable diaper, a sanitary napkin, a panty liner, or an incontinence pad, and is particularly preferable as a topsheet of an absorbent article.

In the case where the composite sheet 10 is used as a topsheet of an absorbent article, typically, the first sheet 1 forms a surface (skin-facing surface) that faces the skin of a wearer, and the second sheet 2 forms a surface (non-skin-facing surface) that faces an absorbent member when the absorbent article is worn.

The composite sheet 10 can also be used for uses other than the topsheet of an absorbent article.

Examples of the uses, other than the topsheet, of the composite sheet 10 in an absorbent article include a sheet that is arranged between the topsheet and the absorbent member and also called a second sheet, a sublayer, or the like; and a sheet for forming a leak-proof wall that is also called leak-proof gathers, a leak-proof cuff, or the like (in particular, a sheet for forming an inner wall of the leak-proof wall).

Examples of the uses of the composite sheet 10 other than the uses in an absorbent article include a cleaning sheet, in particular, a cleaning sheet mainly used to absorb a liquid; and a cosmetic sheet for humans.

In the case where the composite sheet 10 is used as a cleaning sheet, the projections 5 of the composite sheet 10 can favorably follow a non-smooth surface that is to be cleaned, and therefore, it is preferable to use the composite sheet 10 with the first sheet 1 side thereof facing the surface to be cleaned.

In the case where the composite sheet 10 is used as a cosmetic sheet, the projections 5 of the composite sheet 10 can follow the skin of a user, provide a massage effect, and absorb an excess of a cosmetic agent (used separately), or sweat, and therefore, it is preferable to use the composite sheet 10 with the first sheet 1 side thereof facing the skin.

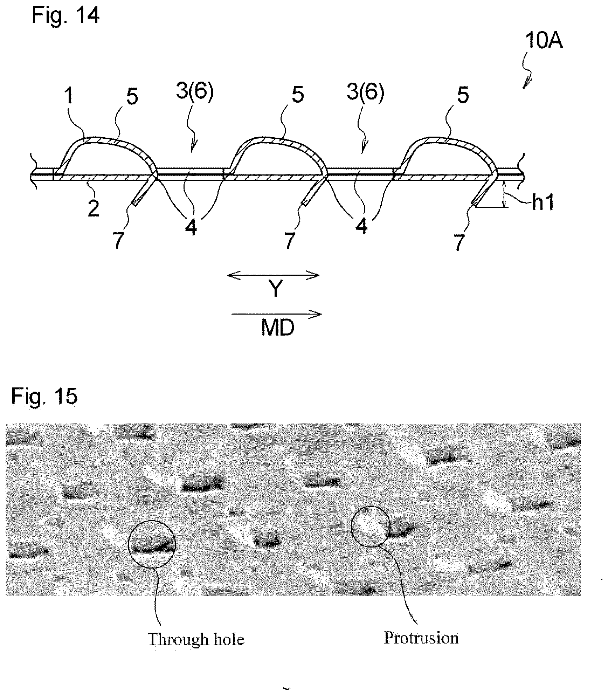

Hereinafter, the composite sheet of the present invention will be described. Figs. 13 and 14 show a composite sheet 10A serving as an embodiment of the composite sheet of the present invention. The composite sheet 10A is produced using the above-described method and apparatus for producing a composite sheet of the present invention. With regard to the composite sheet 10A, different configurations from those of the above-described composite sheet 10 (see Figs. 1 and 2) will be described, and for configurations of the composite sheet 10A that will not be specifically described, descriptions of the composite sheet 10 apply as appropriate.

The composite sheet 10A can be used as a topsheet of an absorbent article including a liquid-retentive absorbent member, the topsheet being arranged on a side closer to the skin of the wearer than the absorbent member, and is a topsheet for an absorbent article. As shown in Figs. 13 and 14, the composite sheet 10A has a plurality of fused portions 4 where the first sheet 1 and the second sheet 2 are fused to each other, at least some portions of the first sheet 1 other than the fused portions 4 form projections 5 protruding toward the opposite side to the second sheet 2 side, and through holes 6 are formed in the fused portions 4. This basic configuration of the composite sheet 10A is the same as that of the composite sheet 10 described above.

In the form shown in Figs. 13 and 14, the composite sheet 10A has a large number of depressions 3 on a first sheet 1-side surface thereof, the depressions 3 being located between the projections 5 both in the direction X and in the direction Y, and a fused portion 4 having a through hole 6 is formed at the bottom of each depression 3. When the composite sheet 10A is viewed as a whole, the first sheet 1-side surface of the composite sheet 10A has a sharp projecting-and-depressed shape constituted by the depressions 3 and the projections 5, while a second sheet 2-side surface of the composite sheet 10A is flat, or is a substantially flat surface that is relatively gently undulating compared with the first sheet 1-side surface.

The composite sheet 10A is characterized in that protrusions 7 are formed on its surface (second sheet 2-side surface) opposite to the surface (first sheet 1-side surface) on which the projections 5 are formed, the protrusions 7 starting from the plurality of fused portions 4, respectively, and protruding in a direction away from the second sheet 2. Typically, the same number of protrusions 7 as the number of the plurality of through holes 6 formed in the composite sheet 10A are formed, or fewer multiple protrusions 7 compared with the number of through holes 6 are formed.

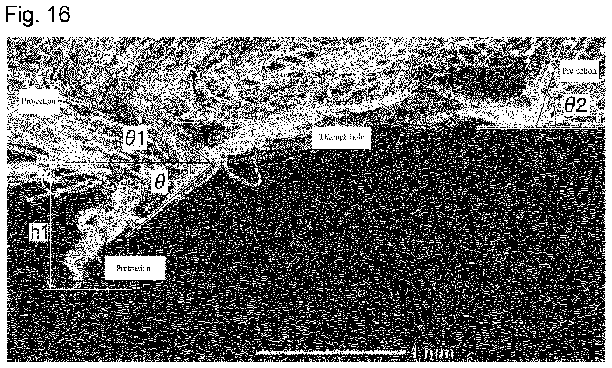

Fig. 15 shows a photograph of the second sheet-side surface of a composite sheet having the same basic configuration as the composite sheet 10A. In Fig. 15, scattered black portions are through holes 6, and white portions extending from an edge (left edge in Fig. 15) of the respective black portions are protrusions 7. Fig. 16 shows an electron micrograph (observation magnification: 30x) of a through hole 6 and a protrusion 7 at a peripheral edge portion the through hole 6, of the composite sheet shown in Fig. 15.

The protrusions 7 are formed at peripheral edge portions of the respective through holes 6.

In the composite sheet 10A shown in Figs. 13 and 14, the protrusions 7 are formed at peripheral edge portions of the respective through holes 6, and are formed on one side (left side in Figs. 13 and 14), and not on the other side (right side in Figs. 13 and 14), with respect to a single through hole 6 in a predetermined one direction (direction Y). The same holds true for the photographs in Figs. 15 and 16.

The protrusions 7 are formed when the composite sheet 10A is produced in the same manner as the composite sheet 10 described above. One of the main factors in forming of the protrusions 7 is presumably that, as the ultrasonic horn used in the ultrasonic-applying step, an ultrasonic horn is used in which, like the ultrasonic horn 42 shown in Figs. 6 to 8, a groove extending along the rotating shaft of the projecting-and-depressed shape roller is formed in the vibration-applying surface. More specifically, as described above and as shown in Fig. 7, while the object to be fused (stack of the first sheet 1 and the second sheet 2) is conveyed in the MD, the object to be fused is sandwiched between the leading end faces 35c of the projections 35 of the projecting-and-depressed shape roller 31 and the vibration-applying surface 42t, in which the groove 46 is formed, of the ultrasonic horn 42, and ultrasonic vibration is applied to the object to be fused. Then, stress that is generated when the object to be fused is pressed is concentrated on the corner 46c located on the forward side (downstream side) in the MD, of the pair of corners 46c that are located in front of and behind the opening 46d of the groove 46 in the MD, and a shear force thus acts on the object to be fused via this corner 46c. It is presumed that this shear force breaks portions of the object to be fused that are in contact with the corner 46c located on the forward side in the MD, the broken portions are then moved to the rearward side (upstream side) in the MD due to the rotation of the projecting-and-depressed shape roller 31, and consequently, through holes 6 are formed, and protrusions 7 are also formed on the rearward end side of the peripheral edge portions of the through holes 6 in the MD.

Therefore, basically, as shown in Figs. 13 and 14, the protrusions 7 are formed on only the rearward end side (upstream side) of the peripheral edge portions of the respective through holes 6 in the machine direction (MD) during the production of the composite sheet 10A, and not on the forward side (downstream side) in the MD Note that, in the photographs shown in Figs. 15 and 16 as well, the left-to-right direction is the MD, and the protrusions are formed on only the rearward side (downstream side) of the peripheral edge portions of the respective through holes in the MD, as in Figs. 13 and 14.

The composite sheet 10A has the plurality of protrusions 7 on the surface opposite to the surface on which the projections 5 are formed. Thus, in the case where the composite sheet 10A is used as a topsheet of an absorbent article, if the composite sheet 10A is arranged such that its surface (first sheet 1-side surface) on which the projections 5 are formed constitutes the skin-facing surface (surface that faces the skin of the wearer of the absorbent article), and the surface (second sheet 2-side surface) opposite to the surface on which the projections 5 are formed constitutes the non-skin-facing surface, when the composite sheet 10A is compressed in the thickness direction by, for example, the body pressure of the wearer when the absorbent article is worn, the protrusions 7 can function as cushions. Accordingly, the cushioning properties of the topsheet are improved, and hence the wearing comfort of the absorbent article can be improved.

Moreover, in an absorbent article in which the composite sheet 10A serving as a topsheet is arranged as described above, the protrusions 7 have the effect of increasing the frictional force between the composite sheet 10A and another member (e.g., a sublayer, which will be described later) arranged on the non-skin-facing surface side of the composite sheet 10A. Therefore, problems such as the topsheet shifting out of position, or peeling off, when the absorbent article is worn are effectively prevented.

As described above, the composite sheet 10A can be produced by using, as the ultrasonic horn used in the ultrasonic-applying step, an ultrasonic horn in which a groove extending along the rotating shaft of the projecting-and-depressed shape roller is formed in the vibration-applying surface. Examples of preferred production conditions are as follows.

- Conveyance speed of the object to be fused (stack of the first sheet 1 and the second sheet 2) in the ultrasonic-applying step: preferably 50 m/minute or greater, or more preferably 100 m/minute or greater, and preferably 400 m/minute or less, or more preferably 300 m/minute or less.
- Angle $\theta 35$ (see Fig. 6) of a corner of the leading end portion of each projection 35 in a cross-sectional view taken along the direction that is perpendicular to the rotating shaft of the projecting-and-depressed shape roller 31 (first roller) (i.e., in a cross-sectional view taken along the MD): preferably 90° or greater, or more preferably 105° or greater, and preferably 135° or less, or more preferably 120° or less.
- Materials of the first sheet 1 and the second sheet 2: a spunbonded nonwoven fabric including core-sheath type composite fibers as constituent fibers, the core-sheath type composite fibers being composed of polyethylene terephthalate (PET) as the core and polyethylene (PE) as the sheath.

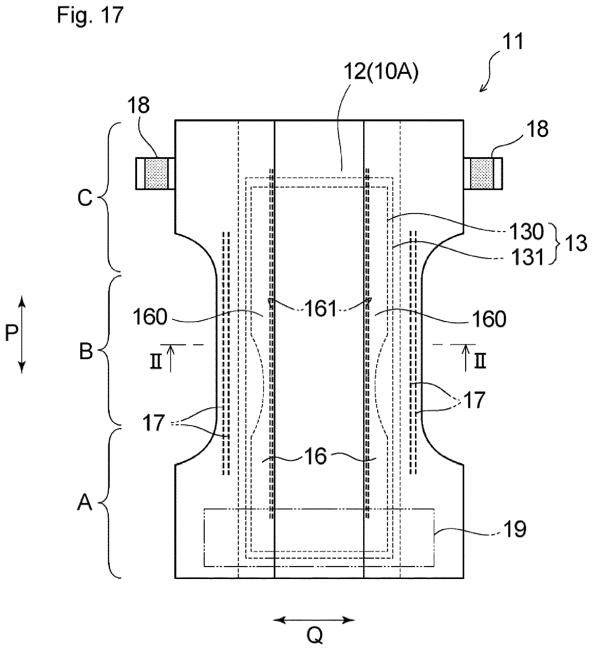

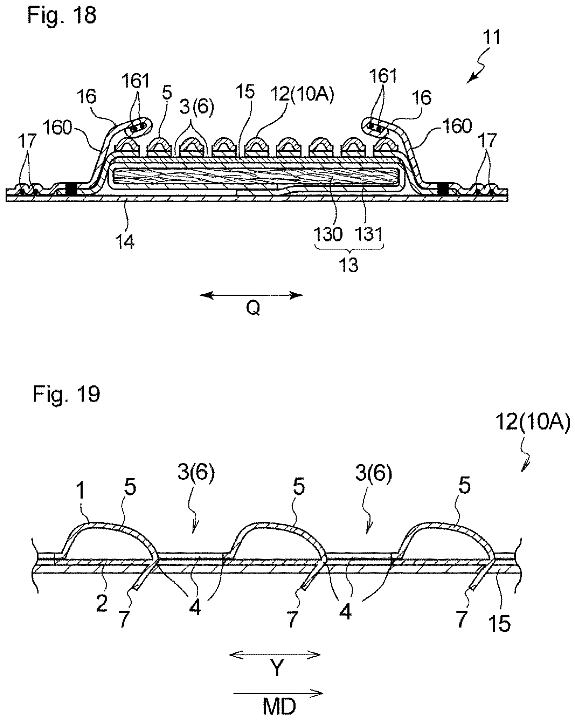

Figs. 17 and 18 show an open type disposable diaper 11 serving as an embodiment of the absorbent article of the present invention. In the diaper 11, the composite sheet 10A is used as a topsheet. The diaper 11 has a longitudinal direction P that corresponds to a front-rear direction of a wearer and a lateral direction Q that is perpendicular to the longitudinal direction P, and includes a liquid-retentive absorbent member 13 and a topsheet 12 that is arranged so as to be located on a side closer to the skin of the wearer than the absorbent member 13. The topsheet 12 is formed of the composite sheet 10A described above.

As shown in Fig. 17, the diaper 11 has a crotch portion B that is to be located on a crotch area of the wearer as well as a front portion A and a rear portion C that extend forward and rearward, respectively, from the crotch portion B. When the diaper 11 is divided into three equal regions in the longitudinal direction X, the front portion A, the crotch portion B, and the rear portion C may correspond to the three regions, respectively. The crotch portion B has an excretion-section opposing portion that opposes an excretion section such as the penis or the anus of the wearer when the diaper 11 is worn, and the excretion-section opposing portion is usually located in or near a middle portion of the diaper 11 in the longitudinal direction P.

As shown in Fig. 18, in the diaper 11, the topsheet 12, a liquid-permeable sublayer 15, and the liquid-retentive absorbent member 13 are stacked in that order from the side closer to the skin of the wearer. More specifically, the diaper 11 includes: the absorbent member 13; the topsheet 12 that is arranged on the skin-facing surface side of the absorbent member 13 and superposed on the absorbent member 13 so as to be located closer to the skin of the wearer than the absorbent member 13; a backsheet 14 that is arranged on the non-skin-facing surface side of the absorbent member 13 and superposed on the absorbent member 13 so as to be located farther from the skin of the wearer than the absorbent member 13; and the sublayer 15 that is provided between the top 12 and the absorbent member 13.

As used herein, the term "skin-facing surface" refers to a surface of the absorbent article or a constituent member (e.g., the absorbent member) thereof that is provided to face the skin of the wearer, or in other words, that is provided on the side relatively close to the skin of the wearer, when the absorbent article is worn, and the term "non-skin-facing surface" refers to a surface of the absorbent article or a constituent member thereof that is provided to face the side (garment side) opposite to the skin of the wearer, or in other words, the side relatively far from the skin of the wearer, when the absorbent article is worn. Note that the expression "when the absorbent article is worn" as used herein means a state in which a normal and properly worn position is maintained, and excludes a case in which the absorbent article is shifted out of its properly worn position.

The topsheet 12 and the backsheet 14 each have larger dimensions than the sublayer 15 and the absorbent member 13, which are provided between the two sheets 2 and 14, and form the outer shape of the diaper 11 in its flat-out, uncontracted state such as that shown in Fig. 17.

The absorbent member 13 has a shape that is elongated in the longitudinal direction P, and extends from the front portion A to the rear portion C. The absorbent member 13 includes a liquid-retentive absorbent core 130 and a core-wrap sheet 131 that covers the outer surface of the absorbent core 130. The absorbent core 130 is typically formed of fiber wads composed mainly of hydrophilic fibers such as wood pulp, and furthermore, water-absorbent polymer particles may be supported on the fiber wads. The core-wrap sheet 131 is typically formed of paper, a nonwoven fabric, or the like.

As the backsheet 14, various types of sheets conventionally used in absorbent articles of this type can be used without particular limitation, and, for example, a resin film, or a laminate of a resin film and a nonwoven fabric or the like, can be used.

The sublayer 15 has the functions of improving the liquid permeability from the topsheet 12 to the absorbent member 13, reducing the return of a liquid absorbed by the absorbent member 13 to the topsheet 12, and the like, and covers substantially the entire region of the skin-facing surface of the absorbent member 13. As the sublayer 15, a hydrophilic and liquid-permeable sheet can be used, and specific examples thereof include paper, woven fabrics, and nonwoven fabrics. Nonwoven fabrics are particularly preferable in view of their relatively high strength and excellent flexibility.

The topsheet 12, the sublayer 15, the absorbent member 13 (the absorbent core 130 and the core-wrap sheet 131), and the backsheet 14 are joined to each other using a known joining means such as an adhesive.

As shown in Figs. 17 and 18, the diaper 11 includes a pair of leak-proof cuffs 16 that are arranged along two end portions of the absorbent member 13 in the lateral direction Q and configured to stand up toward the skin of the wearer in at least the crotch portion B when the diaper 11 is worn. Each leak-proof cuff 16 includes a liquid-resistant or water-repellent and air-permeable leak-proof sheet 160. One end side of the leak-proof sheet 160 in the lateral direction Q is fixed to another member (e.g., ) to form a fixed end portion, and the other end side of the leak-proof sheet 160 in the lateral direction Q is not fixed to any other member and forms a free end portion. An elastic member 161 for forming the leak-proof cuff is provided in the free end portion of the leak-proof sheet 160, the elastic member 161 being fixed while being stretched in the longitudinal direction P, and therefore being able to stretch and contract in the longitudinal direction P. When the diaper 11 is worn, due to the contraction force of the elastic members 161, in at least the crotch portion B, the free end portion side of each leak-proof sheet 160 stands up toward the wearer, with the fixed end portion serving as a stand-up base end, and thus, the pair of leak-proof cuffs 16 stand up, thereby preventing excreta such as urine from flowing out in the lateral direction Q. As the leak-proof sheets 160, various types of sheets that are used as the materials of leak-proof cuffs in absorbent articles of this type can be used without particular limitation, and a liquid-resistant or water-repellent and air-permeable material is preferable. For example, a single- or multi-layer water-repellent nonwoven fabric, or a laminate material of a resin film and a nonwoven fabric or the like, can be used.

As shown in Fig. 17, in each of left and right leg portions that are to be located on the circumferences of the legs of the wearer, a filamentous elastic member 17 is fixed between the leak-proof sheet 160 and the backsheet 14 while being stretched in the longitudinal direction P. Thus, when the diaper 11 is worn, a pair of leg gathers are formed in the leg portions due to the contraction of the elastic members 17. The topsheet 12, the sublayer 15, the backsheet 14, the absorbent member 13, the leak-proof sheets 160, and the elastic members 161 are joined to each other using a known joining means such as a hot-melt adhesive.

As shown in Fig. 17, a pair of pieces of fastening tape 18 are provided in both lateral side edge portions extending in the longitudinal direction P, of the rear portion C of the diaper 11. The pieces of fastening tape 18 are each provided with an attachment portion formed of a male member of a mechanical hook-and-loop fastener. Moreover, an attachment target region 19 formed of a female member of a mechanical hook-and-loop fastener is formed on the non-skin-facing surface of the front portion A of the diaper 11. The attachment target region 19 is formed by joining and fixing a female member of a mechanical hook-and-loop fastener to the non-skin-facing surface of the backsheet 14 that forms the non-skin-facing surface of the front portion A, using a known joining means such as, for example, an adhesive or a heat seal, so that the attachment portions of the pieces of fastening tape 18 can be detachably attached to the attachment target region 19.

The diaper 11 is characterized in that the topsheet 12 is the composite sheet 10A described above. More specifically, as shown in Fig. 19, the topsheet 12 has a plurality of fused portions 4 where the first sheet 1 that forms the skin-facing surface and the second sheet 2 that forms the non-skin-facing surface are fused to each other, at least some portions of the first sheet 1 other than the fused portions 4 form projections 5 protruding toward the opposite side to the second sheet 2 side, and through holes 6 are formed in the fused portions 4. In addition, protrusions 7 are formed on the non-skin-facing surface (second sheet 2-side surface) of the topsheet 12, the protrusions 7 starting from the plurality of fused portions 4, respectively, and protruding in a direction away from the second sheet 2.

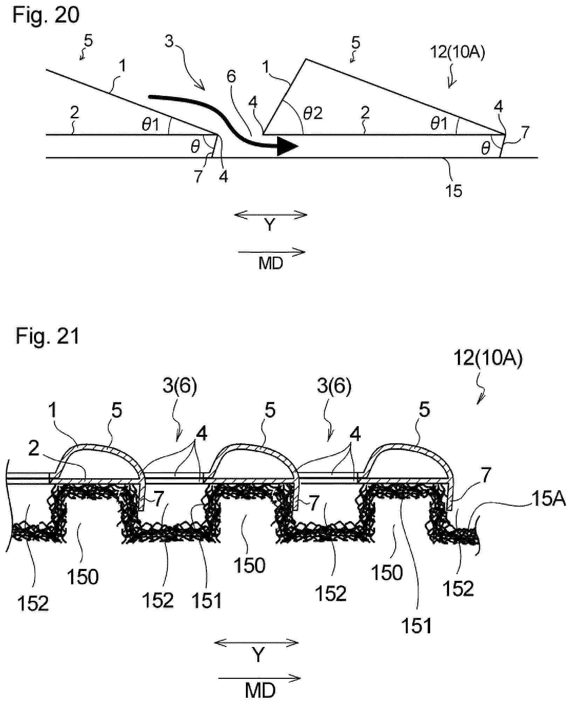

Because of the effect exhibited by the composite sheet 10A due to the above-described protrusions 7, the topsheet 12 has excellent cushioning properties and is unlikely to cause problems such as shifting out of position or peeling off. Accordingly, the diaper 11 including the topsheet 12 (composite sheet 10A) has excellent wearing comfort. From the viewpoint of ensuring that the effect of the protrusions 7 is exhibited even more reliably, it is preferable that, referring to Fig. 19 and also to Fig. 20, which is a more schematic representation of Fig. 19, the angle θ between each protrusion 7 and a portion of the second sheet 2 that is adjacent to a corresponding through hole 6 via the position where the protrusion 7 is formed is less than 90°. If the angle θ exceeds 90°, the protrusion 7 may extend toward the through hole 6, and the through hole 6 and the protrusion 7 may thus overlap each other in the thickness direction. In contrast, when the angle θ is less than 90°, the protrusion 7 does not extend toward the through hole 6 and overlaps a corresponding projection 5 in the thickness direction.

Although the angle θ is preferably less than 90°, an excessively small angle θ may make the effect of the protrusions 7 unlikely to be exhibited. In view of this, the lower limit of the angle θ is preferably 5° or greater, and more preferably 10° or greater.

As described above and as shown in Fig. 19, the protrusions 7 are formed at the peripheral edge portions of the respective through holes 6, and are formed on one side, and not on the other side, with respect to a single through hole 6 in the predetermined one direction Y. The "predetermined one direction" as used herein is not particularly limited, and may be the longitudinal direction P or the lateral direction Q of the diaper 11, for example.

Referring to Fig. 20, when an angle formed on one side with respect to the through hole 6 (in Fig. 20, the left side of the through hole 6) by the first sheet 1 extending in a direction away from the second sheet 2 while starting from the one side of the fused portion 4 where the protrusion 7 is formed, and thereby forming a projection 5 and the second sheet 2 extending from the position where the protrusion 7 is formed toward the opposite side to the through hole 6 is represented by θ1, and

an angle formed on the other side with respect to the through hole 6 (in Fig. 20, the right side of the through hole 6) by the first sheet 1 extending in a direction away from the second sheet 2 while starting from the other side of the fused portion 4 where the protrusion 7 is not formed, and thereby forming another projection 5 and the second sheet 2 extending from the fused portion 4 toward the opposite side to the through hole 6 is represented by θ2, it is preferable that a magnitude relationship of θ1<θ2 is established.

With this configuration in which the angles θ1 and θ2, which are formed by the second sheet 2 and two projections 5 (the first sheet 1) that are adjacent to each other across a depression 3 (a through hole 6), are different from each other, or in other words, inclination angles of the two projections 5 are different from each other, if the wearer of the diaper 11 excretes a stool or the like toward the skin-facing surface of the topsheet 12 (composite sheet 10A), the excreta is likely to be accommodated in a gap between the topsheet 12 and the sublayer 15 through a route indicated by the thick arrow in Fig. 20, and thus, the excreta-absorption performance and the leak-proof performance of the diaper 11 can be improved even more. The projection 5 having the relatively small angle θ1 allows excreta such as a stool to slide down relatively smoothly, and can function as a guide for introducing the excreta in the depression 3 into the through hole 6. On the other hand, the projection 5 having the relatively large angle θ2 can function as a leak-proof wall for preventing the excreta in the depression 3 from moving in a plane direction on the topsheet 12. Accordingly, the excreta in the depression 3 located between these two projections 5 is likely to follow the route indicated by the thick arrow in Fig. 20 and be accommodated in the gap between the topsheet 12 and the sublayer 15.

Moreover, with a configuration in which the magnitude relationship of θ1<θ2 is established, and furthermore, the angle θ relating to the protrusions 7 is less than 90° as described above, a gap that can accommodate a sufficient amount of excreta is more likely to be formed between the topsheet 12 and another member (the sublayer 15 in the form shown) that is arranged on the non-skin-facing surface side of the topsheet 12, and this ensures that the effect of the magnitude relationship of θ1<θ2 is exhibited even more reliably.

Provided that the angle θ1 is smaller than the angle θ2, the angle θ1 is preferably 10° or greater, or more preferably 20° or greater, and preferably 80° or less, or more preferably 70° or less.

Provided that the angle θ2 is larger than the angle θ1, the angle θ2 is preferably 45° or greater, or more preferably 60° or greater, and preferably 80° or less, or more preferably 70° or less.

The angles θ, θ1, and θ2 are each measured using the following method.

Method for Measuring Angles θ, θ1, and θ2

The composite sheet 10A (topsheet 12) to be measured is cut in the thickness direction along the machine direction (MD) during the production thereof. The cut surface is imaged using an SEM (e.g., model: JCM-6000Plus, manufactured by JEOL Ltd.) or a microscope (e.g., model: VHX-1000, manufactured by KEYENCE CORPORATION) at an appropriate magnification. The angles θ, θ1, and θ2 are each measured based on the captured image. To cut the composite sheet 10A, the cutting is performed such that a cutting line created by the cutting extends in the MD while passing through apexes of projections 5. When cutting the composite sheet 10A using a razor blade, if the pressure of the razor blade significantly deforms the projecting-and-depressed shape structure or the protrusions of the sheet, the sheet is dipped in liquid nitrogen and then cut immediately.

The angles θ1 and θ2 are measured using the following procedure. First, in the image captured using the SEM or the microscope, the apex of a single projection 5 (i.e., a portion of the projection 5 that is the farthest from the second sheet 2) and two joints of the single projection 5 that are spaced apart from each other in the MD (i.e., portions of the projection 5 that extend upward from the second sheet 2) are visually identified, and an imaginary triangle is created by connecting the apex and the two joints with imaginary straight lines. Next, of the two corners corresponding to the two joints of the imaginary triangle, the angle of the corner at which a protrusion 7 is formed is regarded as θ1, and the angle of the corner at which no protrusion 7 is formed is regarded as θ2, and the angles θ1 and θ2 are measured.

The angles θ, θ1, and θ2 are measured for five or more projections 5, and arithmetic mean values of the measured values are used as the angles θ, θ1, and θ2 of the composite sheet 5A.

From the viewpoint of ensuring that the above-described effect of the protrusions 7 is exhibited even more reliably, the number of protrusions 7 per unit area (in a plan view, the area of a square region with sides of 10 mm) on the non-skin-facing surface (second sheet 2-side surface) of the topsheet 12 is preferably 2 or greater, or more preferably 4 or greater, and preferably 20 or less, or more preferably 15 or less.

From the same viewpoint, the protruding height h1 (see Figs. 14 and 19) of the protrusions 7 is preferably 0.3 mm or greater, or more preferably 0.5 mm or greater, and preferably 4 mm or less, or more preferably 2.5 mm or less. The protruding height h1 of the protrusions 7 can be measured using the following method.

Method for Measuring Protruding Height of Protrusions

The measurement method will be described using the above-described diaper 11 as an example. A stack of the composite sheet 10A (topsheet 12) to be measured and another member (sublayer 15) that is adjacent to the non-skin-facing surface of the composite sheet 10A is removed from the diaper 11, and used as a measurement sample. At this time, in the case where the other member is fixed to yet another member other than the composite sheet 10A using an adhesive or through fusion bonding or the like, the composite sheet 10A is removed after releasing the fixation between those members by, for example, spraying cold air onto the fixing portion through cold spraying. This procedure applies to all the measurements described in the specification of the present application. In the case where an elastic member that is stretchable in the MD of the composite sheet 10A, such as the elastic members 161 for forming the leak-proof cuffs and the elastic members 17 for forming the leg gathers, is fixed to the composite sheet 10A, the elastic member is cut so as to prevent the composite sheet 10A from stretching/contracting in the MD.

The measurement sample (stack of the composite sheet 10A and the other member) is stretched to a crease-free state. The measurement sample is then cut in the thickness direction along the machine direction (MD) during the production of the composite sheet 10A. The cut surface is imaged using an SEM (e.g., model: JCM-6000Plus, manufactured by JEOL Ltd.) or a microscope (e.g., model: VHX-1000, manufactured by KEYENCE CORPORATION) at an appropriate magnification. The protruding height h1 of the protrusions 7 of the composite sheet 10A is measured based on the captured image. To cut the measurement sample, the cutting is performed such that a cutting line created by the cutting extends in the MD while passing through apexes of projections 5 of the composite sheet 10A. When cutting the composite sheet 10A using a razor blade, if the pressure of the razor blade significantly deforms the projecting-and-depressed shape structure or the protrusions of the sheet, the sheet is dipped in liquid nitrogen and then cut immediately.

The protruding height h1 of the protrusions 7 is measured using the following procedure. First, in the image captured using the SEM or the microscope, the apex of a protrusion 7 and the non-skin-facing surface of the second sheet 2 (i.e., the surface of the composite sheet 10A opposite to the surface on which the projections are formed) are visually identified. Next, an imaginary normal line that passes through the apex of the protrusion 7 and is perpendicular to the non-skin-facing surface of the second sheet 2 is created. The length of a segment of the imaginary normal line between the apex of the protrusion 7 and the non-skin-facing surface of the second sheet 2 is used as the protruding height h1 of the protrusion 7.

In the diaper 11, as described above and as shown in Fig. 19, the sublayer 15, which is another member including fibers, is arranged on a side (non-skin-facing surface side) that is farther from the skin of the wearer than the topsheet 12, and there may be cases where the protrusions 7 penetrate between the fibers of the sublayer 15. Note that, although Fig. 19 shows the protrusions 7 extending through the sublayer 15 in the thickness direction, the state in which "the protrusions 7 penetrate between the fibers of the sublayer 15" as used herein is not limited to the state shown in Fig. 19.

With the configuration in which the protrusions 7 penetrate between the fibers of the sublayer 15, the frictional force between the topsheet 12 and the other member including fibers (in the form shown, the sublayer 15) that is arranged on the non-skin-facing surface side of the topsheet 12 increases even more, and thus, problems such as the topsheet 12 shifting out of position or peeling off when the diaper 11 is worn can be effectively prevented.

Whether or not a protrusion 7 penetrates between the fibers of the other member including fibers can be confirmed by making a cut at a position on a side of the protrusion 7 and in the vicinity of the protrusion 7 using a cutting tool such as a razor blade, and observing the cut surface using an SEM or a microscope. An example of the position where the composite sheet 10A (topsheet 12) is cut to form such a cut surface is a cutting line indicated by the reference sign CL in Figs. 2 and 13. Note that, when cutting the composite sheet 10A using a razor blade, if the pressure of the razor blade significantly deforms the projecting-and-depressed shape structure or the protrusions of the sheet, the sheet is dipped in liquid nitrogen and then cut immediately.

Moreover, in the case where the protrusions 7 penetrate between the fibers of the other member including fibers, the degree of penetration of a protrusion 7 can be evaluated using, as an indicator, the protruding height of the protrusion 7 from a surface of the other member that faces the composite sheet 10A (in the case of the diaper 11 described above, the skin-facing surface of the sublayer 15), and it can be evaluated that the greater the protruding height, the more deeply the protrusion 7 penetrates into the other member. The "protruding height of the protrusion 7 from a surface of the other member that faces the composite sheet 10A" as used herein can be measured using a method that is in conformity with the Method for Measuring Protruding Height of Protrusions section above, and specifically, a method in which the "non-skin-facing surface of the second sheet 2 (i.e., the surface of the composite sheet 10A opposite to the surface on which the projections are formed)" in the Method for Measuring Protruding Height of Protrusions section above is replaced with the "surface of the other member that faces the composite sheet 10A".

Note that, in the absorbent article of the present invention, there may be cases where another member arranged on a side (non-skin-facing surface side) that is farther from the skin of the wearer than the topsheet 12 (composite sheet 10A) has a projecting-and-depressed shape structure on the surface (skin-facing surface) of the other member that faces the topsheet 12. Fig. 21 shows, as a specific example of such case, a form in which a sublayer 15A having a projecting-and-depressed shape structure is arranged on the non-skin-facing surface side of the topsheet 12. The sublayer 15A has, on its surface that faces the topsheet 12, a plurality of skin-side projections 151 that each protrude toward the topsheet 12 and have an inner space 150, as well as skin-side depressions 152 that are located between the plurality of skin-side projections 151, and the protrusions 7 of the topsheet 12 penetrate into the skin-side depressions 152. The sublayer 15A has the same configuration as the above-described sublayer 15, except that it has the projecting-and-depressed shape structure. This form in which the protrusions 7 of the composite sheet 10A (topsheet 12) penetrate into depressions, such as the skin-side depressions 152, of another member also provides the same effect as that of the above-described form in which the protrusions 7 penetrate between fibers of another member.

It is preferable that the non-skin-facing surface (second sheet 2-side surface) of the topsheet 12 and another member (in the form shown, the sublayer 15) that is arranged on the side farther from the skin of the wearer than the topsheet 12 are joined to each other using an adhesive. With this configuration, the topsheet 12 and the other member are integrated, and thus, problems such as the topsheet 12 shifting out of position or peeling off when the diaper 11 is worn are effectively prevented.

In particular, in the case where the other member is a member that includes fibers like the sublayer 15, and, as described above, the protrusions 7 penetrate between the fibers of the member including fibers, if the non-skin-facing surface of the topsheet 12 and the member including fibers are joined to each other using an adhesive, a synergistic effect of the protrusions 7 and the adhesive can enhance the above-described effect even more.

Any adhesives that can be used to join members to each other in absorbent articles of this type can be used without particular limitation as the adhesive, and an example thereof is a hot-melt adhesive.

Although the present invention has been described based on preferred embodiments thereof, the present invention is not limited in any respect to the foregoing embodiments, and appropriate changes can be made thereto without departing from the gist of the present invention.

For example, in the above-described embodiment, a single groove 46 is formed in the vibration-applying surface 42t. However, a plurality of grooves 46 may also be formed in the vibration-applying surface 42t. In this case, for example, a plurality of grooves 46 each extending in the CD may be arranged intermittently in the MD, or a plurality of grooves 46 each extending in the CD may be arranged intermittently in the CD.

Moreover, configurations included in any one of the foregoing embodiments can be applied to the other embodiments.

For example, the vibration-applying surface 42t (see Fig. 12) of the ultrasonic horn 42D in which the projecting-and-depressed shape portion 48 is formed may have, as shown in Fig. 10, an arc shape that is curved inward away from the rotating shaft of the projecting-and-depressed shape roller 31, in a cross-sectional view taken along the direction that is perpendicular to the rotating shaft (i.e., in a cross-sectional view taken along the MD).

Also, in the case where the vibration-applying surface 42t is formed of the heat storage portion 421 as shown in Fig. 11, a projecting-and-depressed shape portion 48 may be formed in the vibration-applying surface 42t formed of the heat storage portion 421.

Furthermore, although the diaper 11 serving as an embodiment of the absorbent article of the present invention includes the sublayer 15 between the topsheet 12 (composite sheet 10A) and the absorbent member 13, the sublayer 15 may be omitted. In this case, in the diaper 11, the topsheet 12 and the absorbent member 13 are in contact with each other, and the protrusions 7 on the non-skin-facing surface of the topsheet 12 may penetrate into the absorbent member 13, or more specifically, penetrate between the fibers of the core-wrap sheet 131 (another member including fibers).

With regard to the foregoing embodiments of the present invention, the following additional remarks will be further disclosed.

<1> A method for producing a composite sheet that has a plurality of fused portions where a first sheet and a second sheet are fused to each other and in which at least some portions of the first sheet other than the fused portions form projections protruding toward an opposite side to the second sheet side, and through holes are formed in the fused portions,

the method comprising:

a shape-imparting step of, while rotating a projecting-and-depressed shape roller that has a projecting-and-depressed shape on a peripheral surface section thereof, causing the first sheet to follow the peripheral surface section to thereby deform the first sheet into a projecting-and-depressed shape;

a superposing step of holding and conveying the first sheet that has been deformed into the projecting-and-depressed shape on the projecting-and-depressed shape roller and superposing the second sheet on the first sheet that is being conveyed; and

an ultrasonic-applying step of sandwiching the superposed two sheets between projections of the projecting-and-depressed shape roller and a vibration-applying surface of a tip portion of an ultrasonic horn included in an ultrasonic fusing machine, and applying ultrasonic vibration to the sheets,

wherein, in the ultrasonic-applying step, the through holes and the fused portions having the through holes are formed by applying ultrasonic vibration using the ultrasonic horn having a groove which is formed on the vibration-applying surface and which extends along a rotating shaft of the projecting-and-depressed shape roller.

<2> The method for producing a composite sheet as set forth in clause <1>,

wherein, in a cross-sectional view taken along a direction that is perpendicular to the rotating shaft of the projecting-and-depressed shape roller, the groove is defined by a pair of depressed sides that intersect the vibration-applying surface and a depressed bottom that is connected to respective ends of the pair of depressed sides in a longitudinal direction and opposes an opening of the groove, and

corners formed by the depressed sides with the vibration-applying surface are sharp, and the depressed bottom has an arc shape that is curved inward away from the opening in the cross-sectional view.

<3> The method for producing a composite sheet as set forth in clause <1> or <2>,
wherein one or more of the grooves are formed in the vibration-applying surface.
<4> The method for producing a composite sheet as set forth in any one of clauses <1> to <3>,
wherein, in a cross-sectional view taken along a direction that is perpendicular to the rotating shaft of the projecting-and-depressed shape roller, the vibration-applying surface has an arc shape that is curved inward away from the rotating shaft.
<5> The method for producing a composite sheet as set forth in any one of clauses <1> to <4>,
wherein at least one of the first sheet and the second sheet before the ultrasonic vibration is applied to the sheets is preheated.
<6> The method for producing a composite sheet as set forth in clause <5>,
wherein the preheating is a treatment in which the sheet to be preheated (at least one of the first sheet and the second sheet before the ultrasonic vibration is applied to the sheets) is heated to a temperature that is below a melting point of the sheet but is not below a temperature that is 50°C lower than the melting point.
<7> The method for producing a composite sheet as set forth in any one of clauses <1> to <6>,
wherein the vibration-applying surface is heated, and, in the ultrasonic-applying step, ultrasonic vibration is applied using the heated vibration-applying surface and the projections of the projecting-and-depressed shape roller.

<8> The method for producing a composite sheet as set forth in any one of clauses <1> to <7>,
wherein, in the shape-imparting step, in addition to the projecting-and-depressed shape roller, another projecting-and-depressed shape roller that has, on a peripheral surface section thereof, a projecting-and-depressed shape engageable with the projecting-and-depressed shape on the peripheral surface section of the projecting-and-depressed shape roller is used, and the first sheet is deformed into the projecting-and-depressed shape by rotating the two rollers such that an engagement portion between the projecting-and-depressed shapes of the two rollers is formed, and introducing the first sheet into the engagement portion.

<9> The method for producing a composite sheet as set forth in any one of clauses <1> to <7>,

wherein the projecting-and-depressed shape roller is configured to be able to suction the first sheet that has been introduced onto the peripheral surface section of the projecting-and-depressed shape roller, toward the peripheral surface section, and
in the shape-imparting step, the first sheet is deformed into the projecting-and-depressed shape only by a suction force of the projecting-and-depressed shape roller.

<10> The method for producing a composite sheet as set forth in any one of clauses <1> to <9>,
wherein, in the ultrasonic-applying step, an applied-pressure that is applied to the superposed two sheets is preferably 10 N/mm or greater, and more preferably 15 N/mm or greater.

<11> The method for producing a composite sheet as set forth in any one of clauses <1> to <10>,
wherein, in the ultrasonic-applying step, an applied-pressure that is applied to the superposed two sheets is preferably 30 N/mm or less, and more preferably 25 N/mm or less.

<12> The method for producing a composite sheet as set forth in any one of clauses <1> to <11>,
wherein, in the ultrasonic-applying step, a frequency of the ultrasonic vibration that is applied to the superposed two sheets is preferably 15 kHz or higher, and more preferably 20 kHz or higher.

<13> The method for producing a composite sheet as set forth in any one of clauses <1> to <12>,
wherein, in the ultrasonic-applying step, a frequency of the ultrasonic vibration that is applied to the superposed two sheets is preferably 50 kHz or less, and more preferably 40 kHz or less.

<14> The method for producing a composite sheet as set forth in any one of clauses <1> to <13>,
wherein, in the ultrasonic-applying step, an amplitude of the ultrasonic vibration that is applied to the superposed two sheets is preferably 20 $\mu$m or greater, and more preferably 25 $\mu$m or greater.

<15> The method for producing a composite sheet as set forth in any one of clauses <1> to <14>,
wherein, in the ultrasonic-applying step, an amplitude of the ultrasonic vibration that is applied to the superposed two sheets is preferably 50 $\mu$m or less, and more preferably 40 $\mu$m or less.

<16> The method for producing a composite sheet as set forth in any one of clauses <1> to <15>,
wherein an area of a region inside an outer circumferential edge of each fused portion is preferably 0.5 mm$^2$ or greater, and more preferably 1.0 mm$^2$ or greater.

<17> The method for producing a composite sheet as set forth in any one of clauses <1> to <16>,
wherein an area of a region inside an outer circumferential edge of each fused portion is preferably 5.0 mm$^2$ or less, and more preferably 4.0 mm$^2$ or less.

<18> The method for producing a composite sheet as set forth in any one of clauses <1> to <17>,

wherein a ratio of a length (length C) of the fused portions in CD to a length (length D) of the fused portions in MD, the former:the latter, is preferably 1:1 or greater, and more preferably 2:3 or greater, wherein
MD represents a machine direction during production of the composite sheet, and CD represents a direction that is perpendicular to the machine direction.

<19> The method for producing a composite sheet as set forth in any one of clauses <1> to <18>,

wherein a ratio of a length (length C) of the fused portions in CD to a length (length D) of the fused portions in MD, the former:the latter, is preferably 1:3 or less, and more preferably 2:5 or less, wherein
MD represents a machine direction during production of the composite sheet, and CD represents a direction that is perpendicular to the machine direction.

<20> The method for producing a composite sheet as set forth in any one of clauses <1> to <19>,
wherein an opening area of each through hole is preferably 50% or greater, and more preferably 80% or greater, of an area of a region inside an outer circumferential edge of each fused portion.

<21> The method for producing a composite sheet as set forth in any one of clauses <1> to <20>,

wherein an opening area of each through hole is preferably less than 100%, and preferably 95% or less, of an area of a region inside an outer circumferential edge of each fused portion.

<22> The method for producing a composite sheet as set forth in any one of clauses <1> to <21>, wherein the fused portions are formed in an annular shape surrounding the respective through holes.

<23> The method for producing a composite sheet as set forth in any one of clauses <1> to <22>, wherein the fused portions are in film form.

<24> An apparatus for producing a composite sheet that has a plurality of fused portions where a first sheet and a second sheet are fused to each other and in which at least some portions of the first sheet other than the fused portions form projections protruding toward an opposite side to the second sheet side, and through holes are formed in the fused portions,

the apparatus comprising:

a projecting-and-depressed-shape-imparting unit that includes a projecting-and-depressed shape roller having a projecting-and-depressed shape on a peripheral surface section thereof, and that is configured to deform the first sheet into a projecting-and-depressed shape by causing the first sheet to follow the peripheral surface section; and

an ultrasonic-applying unit that includes an ultrasonic fusing machine including an ultrasonic horn and is configured to form the through holes and the fused portions having the through holes by superposing the second sheet on the first sheet that has been deformed into the projecting-and-depressed shape, sandwiching the two sheets between projections of the projecting-and-depressed shape roller and a vibration-applying surface of a tip portion of the ultrasonic horn, and applying ultrasonic vibration to the sheets,

wherein the vibration-applying surface has a groove extending along a rotating shaft of the projecting-and-depressed shape roller.

<25> The apparatus for producing a composite sheet as set forth in clause <24>,

wherein, in a cross-sectional view taken along a direction that is perpendicular to the rotating shaft of the projecting-and-depressed shape roller, the groove is defined by a pair of depressed sides that intersect the vibration-applying surface and a depressed bottom that is connected to respective ends of the pair of depressed sides in a longitudinal direction and opposes an opening of the groove, and

corners formed by the depressed sides with the vibration-applying surface are sharp, and the depressed bottom has an arc shape that is curved inward away from the opening in the cross-sectional view.

<26> The apparatus for producing a composite sheet as set forth in clause <25>, wherein an angle between the vibration-applying surface and the respective depressed sides at the corners is preferably 45° or greater, and more preferably 60° or greater.

<27> The apparatus for producing a composite sheet as set forth in clause <25> or <26>, wherein an angle between the vibration-applying surface and the respective depressed sides at the corners is preferably 135° or less, and more preferably 120° or less.

<28> The apparatus for producing a composite sheet as set forth in any one of clauses <25> to <27>, wherein the depressed bottom has a curvature of preferably 1 or greater, and more preferably 2 or greater.

<29> The apparatus for producing a composite sheet as set forth in any one of clauses <25> to <28>, wherein the depressed bottom has a curvature of preferably 10 or less, and more preferably 5 or less.

<30> The apparatus for producing a composite sheet as set forth in any one of clauses <24> to <29>, wherein one or more of the grooves are formed in the vibration-applying surface.

<31> The apparatus for producing a composite sheet as set forth in any one of clauses <24> to <30>, including a preheating means for preheating at least one of the first sheet and the second sheet before the ultrasonic vibration is applied to the sheets.

<32> The apparatus for producing a composite sheet as set forth in any one of clauses <24> to <31>, including a horn heating means for heating the vibration-applying surface.

<33> The apparatus for producing a composite sheet as set forth in any one of clauses <24> to <32>, wherein, in a cross-sectional view taken along a direction that is perpendicular to the rotating shaft of the projecting-and-depressed shape roller, the vibration-applying surface has an arc shape that is curved inward away from the rotating shaft.

<34> The apparatus for producing a composite sheet as set forth in clause <33>, wherein the arc-shaped vibration-applying surface is curved along a circular trajectory of leading ends of the projections of the projecting-and-depressed shape roller.

<35> The apparatus for producing a composite sheet as set forth in clause <33> or <34>, wherein, in a cross-sectional view taken along the direction that is perpendicular to the rotating shaft of the projecting-and-depressed shape roller, a leading end face of each of the plurality of projections of the projecting-and-depressed shape roller has a convex shape that is curved outward away from the rotating shaft of the projecting-and-depressed shape roller, and a curvature direction of the leading end face is the same as that of the vibration-applying surface.

<36> The apparatus for producing a composite sheet as set forth in any one of clauses <24> to <35>, wherein the tip portion of the ultrasonic horn includes a heat storage portion fixed to a metal main body portion of the ultrasonic horn, and the vibration-applying surface is formed of the heat storage portion.

<37> The apparatus for producing a composite sheet as set forth in clause <36>, wherein the heat storage portion has a thickness of preferably 5 μm or greater, and more preferably 10 μm or greater.

<38> The apparatus for producing a composite sheet as set forth in clause <36> or <37>, wherein the heat storage portion has a thickness of preferably 100 μm or less, and more preferably 50 μm or less.

<39> The apparatus for producing a composite sheet as set forth in any one of clauses <24> to <38>, wherein a projecting-and-depressed shape portion is formed on a portion of the vibration-applying surface where the groove is not formed.

<40> The apparatus for producing a composite sheet as set forth in clause <39>, wherein the projecting-and depressed shape portion has a surface roughness of preferably 3.2 μm or greater, and more preferably 6.3 μm or greater, in terms of arithmetic mean roughness Ra.

<41> The apparatus for producing a composite sheet as set forth in clause <39> or <40>, wherein the projecting-and-depressed shape portion has a surface roughness of preferably 25 μm or less, and more preferably 12.5 μm or less, in terms of arithmetic mean roughness Ra.

<42> A composite sheet that can be used as a topsheet of an absorbent article including a liquid-retentive absorbent member, the topsheet being arranged on a side closer to the skin of a wearer than the absorbent member,

wherein the composite sheet has a plurality of fused portions where a first sheet and a second sheet are fused to each other, at least some portions of the first sheet other than the fused portions form projections protruding toward an opposite side to the second sheet side, and through holes are formed in the fused portions, and protrusions are formed on a surface opposite to a surface on which the projections are formed, the protrusions starting from the plurality of fused portions, respectively, and protruding in a direction away from the second sheet.

<43> The composite sheet as set forth in clause <42>, wherein the protrusions are formed at peripheral edge portions of the respective through holes, and an angle between each protrusion and a portion of the second sheet that is adjacent to a corresponding through hole via a position where the protrusion is formed is less than 90°.

<44> The composite sheet as set forth in clause <42> or <43>,

wherein the protrusions are formed at peripheral edge portions of the respective through holes, and are formed on one side, and not on another side, with respect to the through holes in a predetermined one direction, and a magnitude relationship of θ1<θ2 is established,
wherein θ1 represents an angle formed by: the first sheet extending in a direction away from the second sheet while starting from the one side of each fused portion where a corresponding protrusion is formed, and thereby forming a corresponding projection and the second sheet extending from a position where the protrusion is formed toward an opposite side to the through hole, and
θ2 represents an angle formed by: the first sheet extending in a direction away from the second sheet while starting from the other side of the fused portion where the protrusion is not formed, and thereby forming another corresponding projection; and the second sheet extending from the fused portion toward an opposite side to the through hole..

<45> An absorbent article having a longitudinal direction that corresponds to a front-rear direction of a wearer and a lateral direction that is perpendicular to the longitudinal direction, the absorbent article including a liquid-retentive absorbent member and a topsheet that is arranged on a side closer to the skin of the wearer than the absorbent member,

wherein the topsheet has a plurality of fused portions where a first sheet that forms a skin-facing surface and a second sheet that forms a non-skin-facing surface are fused to each other, at least some portions of the first sheet other than the fused portions form projections protruding toward an opposite side to the second sheet side, and through holes are formed in the fused portions, and

protrusions are formed on the non-skin-facing surface of the topsheet, the protrusions starting from the plurality of fused portions, respectively, and protruding in a direction away from the second sheet.

<46> The absorbent article as set forth in clause <45>,
wherein the protrusions are formed at peripheral edge portions of the respective through holes, and an angle between each protrusion and a portion of the second sheet that is adjacent to a corresponding through hole via a position where the protrusion is formed is less than 90°.
<47> The absorbent article as set forth in clause <45> or <46>,

wherein the protrusions are formed at peripheral edge portions of the respective through holes, and are formed on one side, and not on another side, with respect to the through holes in a predetermined one direction, and a magnitude relationship of $\theta1<\theta2$ is established,
wherein $\theta1$ represents an angle formed by the first sheet extending in a direction away from the second sheet while starting from the one side of each fused portion where a corresponding protrusion is formed, and thereby forming a corresponding projection and the second sheet extending from a position where the protrusion is formed toward an opposite side to the through hole, and
$\theta2$ represents an angle formed by the first sheet extending in a direction away from the second sheet while starting from the other side of the fused portion where the protrusion is not formed, and thereby forming another corresponding projection and the second sheet extending from the fused portion toward an opposite side to the through hole.

<48> The absorbent article as set forth in any one of clauses <45> to <47>,
wherein another member including fibers is arranged on a side farther from the skin of the wearer than the topsheet, and the protrusions penetrate between the fibers of the other member.
<49> The absorbent article as set forth in any one of clauses <45> to <48>,
wherein the non-skin-facing surface of the topsheet and another member arranged on a side farther from the skin of the wearer than the topsheet are joined to each other using an adhesive.

Hereinafter, the present invention will be described in greater detail using examples. However, the present invention is not limited to the following examples.

Example 1

A composite sheet having the same basic configuration as the above-described composite sheet 10A, or in other words, "a composite sheet that has a plurality of fused portions where a first sheet and a second sheet are fused to each other and in which at least some portions of the first sheet other than the fused portions form projections protruding toward an opposite side to the second sheet side, through holes are formed in the fused portions, and protrusions are formed on a surface opposite to a surface on which the projections are formed, the protrusions starting from the plurality of fused portions, respectively, and protruding in a direction away from the second sheet" was produced using the same method as the above-described method for producing the composite sheet 10, and the thus produced composite sheet was used as a composite sheet of Example 1.

Comparative Example 1

A composite sheet was produced in the same manner as in the method for producing the composite sheet of Example 1, except that, as the ultrasonic horn used in the ultrasonic-applying step, an ultrasonic horn was used in which no groove extending along the rotating shaft of the projecting-and-depressed shape roller was formed in the vibration-applying surface.

Evaluation Tests

The frictional force and the cushioning properties of the composite sheets of the example and the comparative example were evaluated using the following methods. Table 1 below shows the results.

Method for Evaluating Frictional Force

An air-through nonwoven fabric (having a 20 cm long and 20 cm wide rectangular shape in a plan view) is fixed to a horizontal surface. A composite sheet to be evaluated (having a 5 cm long and 5 cm wide rectangular shape in a plan view) is placed on the air-through nonwoven fabric, with the surface of the composite sheet opposite to the surface on which the projections are formed facing the air-through nonwoven fabric. Furthermore, a 100 g weight is placed on a central portion of the surface (upper surface) of the composite sheet on which the projections are formed so that a load of 0.2 kPa is applied to the composite sheet. Then, the composite sheet under the load is pulled in one direction and moved on the air-through nonwoven fabric at a speed of 300 mm/min, during which the maximum load is measured using a tensile tester. The highest value of the measured values is used as the frictional force (unit: N) of the composite sheet to be evaluated. The higher the numerical value of the frictional force, the less smoothly the composite sheet slides, and it can be evaluated that the composite sheet is less likely to shift out of position or peel off when used as, for example, the topsheet of an absorbent article. For example, "TENSILON (registered trademark), model: RTG-1310, manufactured by A&D Company, Limited" or "AUTOGRAPH (registered trademark), model: AG-X, manufactured by Shimadzu Corporation" can be used as the tensile tester.

Method for Evaluating Cushioning Properties

Work of compression (WC) is used as the indicator for evaluating the cushioning properties. It can be evaluated that the higher the numerical value of the WC, the better the cushioning properties of the evaluation target. The WC is measured using the following procedure.

A 100 mm $\times$ 100 mm square-shaped piece is cut out from the evaluation target (composite sheet) and used as a test specimen. The test specimen is placed on a test stand of a compression tester (model: KES FB3-AUTO-A, manufactured by Kato Tech Co., Ltd.), and the test specimen is compressed in its thickness direction by pressing the test specimen from the side on which the projections are formed (the first sheet side) using a pressure-applying tool included in the compression tester. The above-described compression of the test specimen is performed under the conditions of a compression area of 2 cm$^2$, a compression speed of 0.02 mm/second, and a maximum compression load of 4.9 kPa (50 gf/cm$^2$). The WC (unit: gf•cm/cm$^2$) of the test specimen is calculated using an equation (1) below. In the equation (1) below, $T_m$ represents the thickness under a load of 4.9 kPa (50 gf/cm$^2$), $T_o$ represents the thickness under a load of 49 Pa (0.5 gf/cm$^2$), and $P_a$ represents the load during the measurement (compression process). For each evaluation target, the WC is measured three times, and an arithmetic mean value of the thus obtained three measured values is used as the WC of the evaluation target.

[Math. 1]

$$WC = \int_{T_0}^{T_m} P_a dT \quad \cdot\cdot\cdot \quad (1)$$

Table 1

|  | Ex. 1 | Com. Ex. 1 |
|---|---|---|
| Type of first sheet | Air-through nonwoven fabric | Air-through nonwoven fabric |
| Basis weight of first sheet (g/mL) | 23 | 23 |
| Type of second sheet | Air-through nonwoven fabric | Air-through nonwoven fabric |
| Basis weight of second sheet (g/m$^2$) | 18 | 18 |
| Whether or not through holes located in fused portions were present | Present | Present |
| Whether or not protrusions were present on surface opposite to surface on which projections were formed | Present | Absent |
| Number of protrusions per unit area (10 mm $\times$ 10 mm) | 4 | 0 |
| Protruding height h1 of protrusions (mm) | 1.29 | - |
| Angle θ (°) | 20 | - |
| Frictional force (N) | 1.3 | 0.9 |

(continued)

|  | Ex. 1 | Com. Ex. 1 |
|---|---|---|
| Work of compression (WC) (gf•cm/cm$^2$) | 0.44 | 0.38 |

As shown in Table 1, it can be seen that, since the composite sheet of Example 1 had the protrusions on the surface opposite to the surface on which the projections were formed, this composite sheet had a greater frictional force, compared with the composite sheet of Comparative Example 1, which had no protrusions, and therefore, the composite sheet of Example 1 was unlikely to shift out of position or peel off when arranged superposed on the surface of another member, and also had a great WC and therefore excellent cushioning properties.

Industrial Applicability

The present invention provides a method and an apparatus for producing a composite sheet with which, during the production of a composite sheet in which through holes are formed in fused portions, the fused portions and the through holes can be formed in one step.

A composite sheet of the present invention has excellent cushioning properties and is unlikely to shift out of position or peel off when arranged superposed on the surface of another member.

An absorbent article of the present invention includes a composite sheet that can exhibit the above-described effects as a topsheet, and therefore has excellent wearing comfort.

**Claims**

1. A method for producing a composite sheet that has a plurality of fused portions where a first sheet and a second sheet are fused to each other and in which at least some portions of the first sheet other than the fused portions form projections protruding toward an opposite side to the second sheet side, and through holes are formed in the fused portions,

   the method comprising:

   a shape-imparting step of, while rotating a projecting-and-depressed shape roller that has a projecting-and-depressed shape on a peripheral surface section thereof, causing the first sheet to follow the peripheral surface section to thereby deform the first sheet into a projecting-and-depressed shape;
   a superposing step of holding and conveying the first sheet that has been deformed into the projecting-and-depressed shape on the projecting-and-depressed shape roller and superposing the second sheet on the first sheet that is being conveyed; and
   an ultrasonic-applying step of sandwiching the superposed two sheets between projections of the projecting-and-depressed shape roller and a vibration-applying surface of a tip portion of an ultrasonic horn included in an ultrasonic fusing machine, and applying ultrasonic vibration to the sheets,

   wherein, in the ultrasonic-applying step, the through holes and the fused portions having the through holes are formed by applying ultrasonic vibration using the ultrasonic horn having a groove which is formed on the vibration-applying surface and which extends along a rotating shaft of the projecting-and-depressed shape roller.

2. The method for producing a composite sheet according to claim 1,

   wherein, in a cross-sectional view taken along a direction that is perpendicular to the rotating shaft of the projecting-and-depressed shape roller, the groove is defined by a pair of depressed sides that intersect the vibration-applying surface and a depressed bottom that is connected to respective ends of the pair of depressed sides in a longitudinal direction and opposes an opening of the groove, and
   corners formed by the depressed sides with the vibration-applying surface are sharp, and the depressed bottom has an arc shape that is curved inward away from the opening in the cross-sectional view.

3. The method for producing a composite sheet according to claim 1 or 2,
   wherein one or more of the grooves are formed in the vibration-applying surface.

**4.** The method for producing a composite sheet according to any one of claims 1 to 3,
wherein, in a cross-sectional view taken along a direction that is perpendicular to the rotating shaft of the projecting-and-depressed shape roller, the vibration-applying surface has an arc shape that is curved inward away from the rotating shaft.

**5.** The method for producing a composite sheet according to any one of claims 1 to 4,
wherein at least one of the first sheet and the second sheet before the ultrasonic vibration is applied to the sheets is preheated.

**6.** The method for producing a composite sheet according to claim 5,
wherein the preheating is a treatment in which the sheet to be preheated is heated to a temperature that is below a melting point of the sheet but is not below a temperature that is 50°C lower than the melting point.

**7.** The method for producing a composite sheet according to any one of claims 1 to 6,
wherein the vibration-applying surface is heated, and, in the ultrasonic-applying step, ultrasonic vibration is applied using the heated vibration-applying surface and the projections of the projecting-and-depressed shape roller.

**8.** The method for producing a composite sheet according to any one of claims 1 to 7,
wherein, in the shape-imparting step, in addition to the projecting-and-depressed shape roller, another projecting-and-depressed shape roller that has, on a peripheral surface section thereof, a projecting-and-depressed shape engageable with the projecting-and-depressed shape on the peripheral surface section of the projecting-and-depressed shape roller is used, and the first sheet is deformed into the projecting-and-depressed shape by rotating the two rollers such that an engagement portion between the projecting-and-depressed shapes of the two rollers is formed, and introducing the first sheet into the engagement portion.

**9.** The method for producing a composite sheet according to any one of claims 1 to 7,

wherein the projecting-and-depressed shape roller is configured to be able to suction the first sheet that has been introduced onto the peripheral surface section of the projecting-and-depressed shape roller, toward the peripheral surface section, and
in the shape-imparting step, the first sheet is deformed into the projecting-and-depressed shape only by a suction force of the projecting-and-depressed shape roller.

**10.** The method for producing a composite sheet according to any one of claims 1 to 9,
wherein, in the ultrasonic-applying step, an applied-pressure that is applied to the superposed two sheets is 10 N/mm or greater.

**11.** The method for producing a composite sheet according to any one of claims 1 to 10,
wherein, in the ultrasonic-applying step, an applied-pressure that is applied to the superposed two sheets is 30 N/mm or less.

**12.** The method for producing a composite sheet according to any one of claims 1 to 11,
wherein, in the ultrasonic-applying step, a frequency of the ultrasonic vibration that is applied to the superposed two sheets is 15 kHz or higher.

**13.** The method for producing a composite sheet according to any one of claims 1 to 12,
wherein, in the ultrasonic-applying step, a frequency of the ultrasonic vibration that is applied to the superposed two sheets is 50 kHz or less.

**14.** The method for producing a composite sheet according to any one of claims 1 to 13,
wherein, in the ultrasonic-applying step, an amplitude of the ultrasonic vibration that is applied to the superposed two sheets is 20 $\mu$m or greater.

**15.** The method for producing a composite sheet according to any one of claims 1 to 14,
wherein, in the ultrasonic-applying step, an amplitude of the ultrasonic vibration that is applied to the superposed two sheets is 50 $\mu$m or less.

**16.** The method for producing a composite sheet according to any one of claims 1 to 15,

wherein an area of a region inside an outer circumferential edge of each fused portion is 0.5 mm$^2$ or greater.

17. The method for producing a composite sheet according to any one of claims 1 to 16,
wherein an area of a region inside an outer circumferential edge of each fused portion is 5.0 mm$^2$ or less.

18. The method for producing a composite sheet according to any one of claims 1 to 17,

whereina ratio of a length of the fused portions in CD to a length of the fused portions in MD, the former:the latter, is 1:1 or greater, wherein
MD represents a machine direction during production of the composite sheet, and CD represents a direction that is perpendicular to the machine direction.

19. The method for producing a composite sheet according to any one of claims 1 to 18,

wherein a ratio of a length of the fused portions in CD to a length of the fused portions in MD, the former:the latter, is 1:3 or less, wherein
MD represents a machine direction during production of the composite sheet, and CD represents a direction that is perpendicular to the machine direction.

20. The method for producing a composite sheet according to any one of claims 1 to 19,
wherein an opening area of each through hole is 50% or greater of an area of a region inside an outer circumferential edge of each fused portion.

21. The method for producing a composite sheet according to any one of claims 1 to 20,
wherein an opening area of each through hole is less than 100% of an area of a region inside an outer circumferential edge of each fused portion.

22. The method for producing a composite sheet according to any one of claims 1 to 21,
wherein the fused portions are formed in an annular shape surrounding the respective through holes.

23. The method for producing a composite sheet according to any one of claims 1 to 22,
wherein the fused portions are in film form.

24. An apparatus for producing a composite sheet that has a plurality of fused portions where a first sheet and a second sheet are fused to each other and in which at least some portions of the first sheet other than the fused portions form projections protruding toward an opposite side to the second sheet side, and through holes are formed in the fused portions,

the apparatus comprising:

a projecting-and-depressed-shape-imparting unit that includes a projecting-and-depressed shape roller having a projecting-and-depressed shape on a peripheral surface section thereof, and that is configured to deform the first sheet into a projecting-and-depressed shape by causing the first sheet to follow the peripheral surface section; and
an ultrasonic-applying unit that includes an ultrasonic fusing machine including an ultrasonic horn and is configured to form the through holes and the fused portions having the through holes by superposing the second sheet on the first sheet that has been deformed into the projecting-and-depressed shape, sandwiching the two sheets between projections of the projecting-and-depressed shape roller and a vibration-applying surface of a tip portion of the ultrasonic horn, and applying ultrasonic vibration to the sheets,

wherein the vibration-applying surface has a groove extending along a rotating shaft of the projecting-and-depressed shape roller.

25. The apparatus for producing a composite sheet according to claim 24,

wherein, in a cross-sectional view taken along a direction that is perpendicular to the rotating shaft of the projecting-and-depressed shape roller, the groove is defined by a pair of depressed sides that intersect the vibration-applying surface and a depressed bottom that is connected to respective ends of the pair of depressed sides in a longitudinal direction and opposes an opening of the groove, and
corners formed by the depressed sides with the vibration-applying surface are sharp, and the depressed bottom has an arc shape that is curved inward away from the opening in the cross-sectional view.

26. The apparatus for producing a composite sheet according to claim 25,
    wherein an angle between the vibration-applying surface and the respective depressed sides at the corners is 45° or greater.

27. The apparatus for producing a composite sheet according to claim 25 or 26,
    wherein an angle between the vibration-applying surface and the respective depressed sides at the corners is 135° or less.

28. The apparatus for producing a composite sheet according to any one of claims 25 to 27,
    wherein the depressed bottom has a curvature of 1 or greater.

29. The apparatus for producing a composite sheet according to any one of claims 25 to 28,
    wherein the depressed bottom has a curvature of 10 or less.

30. The apparatus for producing a composite sheet according to any one of claims 24 to 29,
    wherein one or more of the grooves are formed in the vibration-applying surface.

31. The apparatus for producing a composite sheet according to any one of claims 24 to 30, comprising a preheating means for preheating at least one of the first sheet and the second sheet before the ultrasonic vibration is applied to the sheets.

32. The apparatus for producing a composite sheet according to any one of claims 24 to 31, comprising a horn heating means for heating the vibration-applying surface.

33. The apparatus for producing a composite sheet according to any one of claims 24 to 32,
    wherein, in a cross-sectional view taken along a direction that is perpendicular to the rotating shaft of the projecting-and-depressed shape roller, the vibration-applying surface has an arc shape that is curved inward away from the rotating shaft.

34. The apparatus for producing a composite sheet according to claim 33,
    wherein the arc-shaped vibration-applying surface is curved along a circular trajectory of leading ends of the projections of the projecting-and-depressed shape roller.

35. The apparatus for producing a composite sheet according to claim 33 or 34,
    wherein, in a cross-sectional view taken along the direction that is perpendicular to the rotating shaft of the projecting-and-depressed shape roller, a leading end face of each of the plurality of projections of the projecting-and-depressed shape roller has a convex shape that is curved outward away from the rotating shaft of the projecting-and-depressed shape roller, and a curvature direction of the leading end face is the same as that of the vibration-applying surface.

36. The apparatus for producing a composite sheet according to any one of claims 24 to 35,
    wherein the tip portion of the ultrasonic horn includes a heat storage portion fixed to a metal main body portion of the ultrasonic horn, and the vibration-applying surface is formed of the heat storage portion.

37. The apparatus for producing a composite sheet according to claim 36,
    wherein the heat storage portion has a thickness of 5 $\mu$m or greater.

38. The apparatus for producing a composite sheet according to claim 36 or 37,
    wherein the heat storage portion has a thickness of 100 $\mu$m or less.

39. The apparatus for producing a composite sheet according to any one of claims 24 to 38,
    wherein a projecting-and-depressed shape portion is formed on a portion of the vibration-applying surface where the groove is not formed.

40. The apparatus for producing a composite sheet according to claim 39,

wherein the projecting-and depressed shape portion has a surface roughness of 3.2 $\mu$m or greater in terms of arithmetic mean roughness Ra.

41. The apparatus for producing a composite sheet according to claim 39 or 40,
wherein the projecting-and-depressed shape portion has a surface roughness of 25 $\mu$m or less in terms of arithmetic mean roughness Ra.

42. A composite sheet that can be used as a topsheet of an absorbent article including a liquid-retentive absorbent member, the topsheet being arranged on a side closer to the skin of a wearer than the absorbent member,

wherein the composite sheet has a plurality of fused portions where a first sheet and a second sheet are fused to each other, at least some portions of the first sheet other than the fused portions form projections protruding toward an opposite side to the second sheet side, and through holes are formed in the fused portions, and protrusions are formed on a surface opposite to a surface on which the projections are formed, the protrusions starting from the plurality of fused portions, respectively, and protruding in a direction away from the second sheet.

43. The composite sheet according to claim 42,
wherein the protrusions are formed at peripheral edge portions of the respective through holes, and an angle between each protrusion and a portion of the second sheet that is adjacent to a corresponding through hole via a position where the protrusion is formed is less than 90°.

44. The composite sheet according to claim 42 or 43,

wherein the protrusions are formed at peripheral edge portions of the respective through holes, and are formed on one side, and not on another side, with respect to the through holes in a predetermined one direction, and a magnitude relationship of $\theta1<\theta2$ is established,
wherein $\theta1$ represents an angle formed by: the first sheet extending in a direction away from the second sheet while starting from the one side of each fused portion where a corresponding protrusion is formed, and thereby forming a corresponding projection and the second sheet extending from a position where the protrusion is formed toward an opposite side to the through hole, and
$\theta2$ represents an angle formed by: the first sheet extending in a direction away from the second sheet while starting from the other side of the fused portion where the protrusion is not formed, and thereby forming another corresponding projection; and the second sheet extending from the fused portion toward an opposite side to the through hole..

45. An absorbent article having a longitudinal direction that corresponds to a front-rear direction of a wearer and a lateral direction that is perpendicular to the longitudinal direction, the absorbent article comprising a liquid-retentive absorbent member and a topsheet that is arranged on a side closer to the skin of the wearer than the absorbent member,

wherein the topsheet has a plurality of fused portions where a first sheet that forms a skin-facing surface and a second sheet that forms a non-skin-facing surface are fused to each other, at least some portions of the first sheet other than the fused portions form projections protruding toward an opposite side to the second sheet side, and through holes are formed in the fused portions, and
protrusions are formed on the non-skin-facing surface of the topsheet, the protrusions starting from the plurality of fused portions, respectively, and protruding in a direction away from the second sheet.

46. The absorbent article according to claim 45,
wherein the protrusions are formed at peripheral edge portions of the respective through holes, and an angle between each protrusion and a portion of the second sheet that is adjacent to a corresponding through hole via a position where the protrusion is formed is less than 90°.

47. The absorbent article according to claim 45 or 46,

wherein the protrusions are formed at peripheral edge portions of the respective through holes, and are formed on one side, and not on another side, with respect to the through holes in a predetermined one direction, and a magnitude relationship of $\theta1<\theta2$ is established,
wherein $\theta1$ represents an angle formed by the first sheet extending in a direction away from the second sheet while starting from the one side of each fused portion where a corresponding protrusion is formed, and thereby forming a corresponding projection and the second sheet extending from a position where the protrusion is formed toward an opposite side to the through hole, and

θ2 represents an angle formed by the first sheet extending in a direction away from the second sheet while starting from the other side of the fused portion where the protrusion is not formed, and thereby forming another corresponding projection and the second sheet extending from the fused portion toward an opposite side to the through hole.

48. The absorbent article according to any one of claims 45 to 47,
wherein another member including fibers is arranged on a side farther from the skin of the wearer than the topsheet, and the protrusions penetrate between the fibers of the other member.

49. The absorbent article according to any one of claims 45 to 48,
wherein the non-skin-facing surface of the topsheet and another member arranged on a side farther from the skin of the wearer than the topsheet are joined to each other using an adhesive.

Fig. 1

Fig. 2

Fig. 3

Fig. 4

Fig. 5

41

43

44

42 (420)

42t

CD

Fig. 6

Fig. 7

Fig. 8

Fig. 9

Fig. 10

Fig. 11

Fig. 12(a)

Fig. 12(b)

Fig. 13

Fig. 14

Fig. 15

Through hole          Protrusion

Fig. 16

Fig. 17

Fig. 18

Fig. 19

Fig. 20

Fig. 21

**INTERNATIONAL SEARCH REPORT**

| International application No. |
| --- |
| PCT/JP2020/043287 |

A.  CLASSIFICATION OF SUBJECT MATTER
B65H 37/04(2006.01)i; B65H 45/12(2006.01)i; B29C 59/04(2006.01)i; B29C 65/08(2006.01)i
FI: B29C65/08; B29C59/04 Z; B65H45/12; B65H37/04 Z

According to International Patent Classification (IPC) or to both national classification and IPC

B.  FIELDS SEARCHED

Minimum documentation searched (classification system followed by classification symbols)
B65H37/04; B65H45/12; B29C59/04; B29C65/08

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2019-093598 A (KAO CORP.) 20 June 2019 (2019-06-20) claims 1-9, fig. 1-11 | 1-49 |
| A | JP 2019-188802 A (KAO CORP.) 31 October 2019 (2019-10-31) claims 1-12, fig. 1-11 | 1-49 |
| A | JP 2010-115283 A (UNI-CHARM CORP.) 27 May 2010 (2010-05-27) claims 1-7, fig. 1-11 | 1-49 |
| A | JP 6450310 B2 (ZUIKO CORP.) 14 December 2018 (2018-12-14) claims 1-6, fig. 1-8 | 1-49 |
| A | JP 2013-522135 A (TETRA LAVAL HOLDINGS & FINANCE S.A.) 13 June 2013 (2013-06-13) claims 1-13, fig. 1-11 | 1-49 |

☐ Further documents are listed in the continuation of Box C.  ☒ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 23 December 2020 (23.12.2020) | 12 January 2021 (12.01.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT
Information on patent family members

International application No.

PCT/JP2020/043287

| Patent Documents referred in the Report | Publication Date | Patent Family | Publication Date |
|---|---|---|---|
| JP 2019-093598 A | 20 Jun. 2019 | (Family: none) | |
| JP 2019-188802 A | 31 Oct. 2019 | WO 2019/203074 A1<br>TW 202003205 A | |
| JP 2010-115283 A | 27 May 2010 | US 2010/0116409 A1<br>claims 1-7, fig. 1-11<br>WO 2010/055854 A1<br>EP 2359786 A1<br>CN 102209513 A<br>KR 10-2011-0093833 A | |
| JP 6450310 B2 | 14 Dec. 2018 | US 2016/0107377 A1<br>claims 1-7, fig. 1-8<br>WO 2014/200102 A1<br>EP 2990181 A<br>CN 105283300 A | |
| JP 2013-522135 A | 13 Jun. 2013 | US 2013/0008132 A1<br>claims 1-13, fig. 1-11<br>WO 2011/117119 A1<br>EP 2368694 A1<br>CN 102802920 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006175688 A

- JP 2006175689 A